# EUROPEAN PATENT SPECIFICATION

(11) **EP 1 618 377 B1**
(45) Date of publication and mention of the grant of the patent: **27.07.2011**
(21) Application number: 04749731.8
(22) Date of filing: 02.04.2004
(51) Int. Cl.: G01N 33/53, C12Q 1/28

(54) **CHROMOGENIC SUBSTRATE WITH A PH INDICATOR DYE**
CHROMOGENES SUBSTRAT MIT PH-INDIKATORFARBSTOFF
SUBSTRAT CHROMOGENE MUNI D'UN COLORANT INDICATEUR DE PH

(30) Priority: 29.04.2003 US 426169
(43) Date of publication of application: 25.01.2006
(73) Proprietor: NEOGEN CORPORATION, Lansing, MI 48912 (US)
(72) Inventor: MAYER, Brent, A., Lexington, KY 40505 (US)
(74) Representative: Grünecker, Kinkeldey, Stockmair & Schwanhäusser Anwaltssozietät
(86) International application number: PCT/US2004/010356
(87) International publication number: WO 2004/097367

(56) References cited:
- WO-A-93/25905
- WO-A1-99/01768
- US-A- 4 444 879
- US-A- 4 946 776
- US-A- 5 215 885
- US-B1- 6 221 624
- FREY A ET AL: "A stable and highly sensitive 3,3',5,5'-tetramethylbenzidine-based substrate reagent for enzyme-linked immunosorbent assays." JOURNAL OF IMMUNOLOGICAL METHODS. 13 JAN 2000, vol. 233, no. 1-2, 13 January 2000 (2000-01-13), pages 47-56, XP002384514 ISSN: 0022-1759
- KEMP PHILIP ET AL: "Validation of a microtiter plate ELISA for screening of postmortem blood for opiates and benzodiazepines." JOURNAL OF ANALYTICAL TOXICOLOGY. OCT 2002, vol. 26, no. 7, October 2002 (2002-10), pages 504-512, XP009067685 ISSN: 0146-4760
- SHELEF ET AL.: 'Novel selective and non-selective optical detection of microorganisms' LETTERS IN APPLIED MICROBIOLOGY vol. 25, 1997, pages 202 - 206, XP002904880

## Description

### (1) Field of the Invention

The present invention relates to the use of a composition for an enzyme-linked assay which comprises a chromogenic substrate that produces a detectable color with a maximum absorbance at one wavelength in reactions of the assay which contain the enzyme and a pH indicator dye that produces a detectable color with a maximum absorbance at a second wavelength when the pH of the reactions of the assay is changed to terminate the reactions for distinguisting a negative reaction from a false negative reaction in the assay. The chromogenic substrate enables colorimetric detection of a bindable substance in positive reactions of the assay and the pH indicator dye enables negative reactions of the assay to be colorimetrically distinguished from false negative reactions. Preferably, the composition is used in enzyme-linked immunoassays such as ELISAs. Most preferably, the enzyme is a peroxidase. In a further preferred embodiment, the chromogenic substrate is 3,3',5,5'-tetramethylbenzidine (TMB) and the pH indicator dye is preferably selected from the group consisting of cresol red and m-cresol purple.

### (2) Description of Related Art

Enzyme-linked immunosorbent assays (ELISA) are the most commonly used immunoassays for a wide variety of applications in diagnostics, research, food testing, process quality assurance and quality control, and environmental testing. ELISAs utilize a label enzyme and a substrate for the enzyme to produce a detectable signal for quantitating antigens, haptens, or antibodies. The sensitivity of ELISAs depends on the particular label enzyme and substrate combination used. Horseradish peroxidase (HRP) has been found to be well suited for use as a label enzyme in ELISAs because it is highly specific, sensitive, and very stable in catalyzing chromogenic, luminescence, and fluorescence reactions.

To increase sensitivity of peroxidase-linked ELISAs, colorless or slightly colorless chromogenic substrates are used. These chromogenic substrates include 3,3',5.5'-tetramethylbenzidine (TMB), ortho-phenylenediamine dihydrochloride (OPD), and 2,2'- azinobis (3-ethyl-benzothiazoline-6-sulfonic acid) diammonium salt (ABTS). WO 93/25905 discloses an immunoassay method that utilizes a pH indicator for assessing whether a sample was added to the reaction to avoid false negative results U.S. Patent No. 4,503,143 to Gerber et al. discloses ELISA and other immunoassays which use TMB, as the chromogenic substrate. U.S Patent No. 5,206,150 to Tai discloses water-based TMB solutions. Colorless chromogenic substrates reduce background signal which increases the overall sensitivity of the assay. However, a problem associated with using colorless chromogenic substrates is that during dispensing of a colorless substrate into a plurality of transparent tubes or wells of an ELISA plate, it is difficult to determine immediately by visual examination whether the chromogenic substrate had been added to a particular tube or well or not. As a consequence, in some cases the chromogenic substrate may not be added to some tubes or wells or added twice to some tubes or wells.

A seconds problem associated with using colorless chromogenic substrates is determining whether a negative reaction is negative because the sample for the reaction did not contain the analyte being tested for or is negative because one or more reagents necessary for the reaction had been omitted, for example the colorless chromogenic substrate. The second problem is particularly acute in peroxidase-linked ELISAs which includes an acid stop following oxidation of the chromogenic substrate. Because it is not possible to distinguish these false negatives due to omitted sample or acid stop from actual negatives due to absence of the analyte in the sample or other false negatives caused by some other failure in the reaction such as omitted sample or degraded analyte in a sample, the number of negative reactions in an assay is artificially elevated.

A solution to the first problem was disclosed in U.S. Patent No. 6,221,624 B1 to Lihme and Wikborg which describes a pre-stained composition comprising TMB and a visible dye such as phloxine B, m-cresol purple, or the like. The dye is used at a concentration which imparts a color to a solution containing the composition which is visible to the eye. The colored solution enables the user to visually determine that the chromogenic substrate is or has been added to a tube or well of an ELISA plate during pipetting. The dye is preferably selected so as to have no absorbance in the absorbances range under the conditions where the reaction product of the reaction between the chromogenic substrate and the enzyme is to be detected and to have substantially no influence on the enzyme-chromogenic substrate reaction. The preferred dye is phloxine B which is used at a concentration which provides a visible when added to a peroxidase reaction. Most peroxidase-based immunoassays use an acid stop to end peroxidase reactions because the acid stop stabilizes the oxidized product thereby increasing sensitivity of the assay. Phloxine B is rendered colorless when an acid stop is added to the reaction. Therefore, the above composition comprising phloxine B or similar dyes does not enable the user after the reactions have been completed to determine whether a negative reaction is an actual negative caused by an absence of analyte in the sample or a false negative caused by an omission of the chromogenic composition.

The second problem has not been addressed. Therefore, a need remains for a method that will enable actual negative reactions (absence of analyte in the sample) to be distinguished from false negatives caused by omission of particular reaction reagents in peroxidase-linked immunoassays such as ELISAs whilst not interfering with positive reactions.

### SUMMARY OF THE INVENTION

The subject matter of the present invention is defined in the claims. Further prefered embodiments are described herein below. It is provided a composition which comprises a chromogenic substrate with an absorbance at one wavelength and a pH indicator dye with an absorbance at a second wavelength. The composition enables both detection of a bindable substance and confirmation of actual negative reactions in enzyme-linked assays, particularly enzyme-linked immunoassays such as ELISAs. In particular, the chromogenic substrate enables colorimetric detection of the bindable substance in positive reactions of the assay and the pH indicator dye enables actual negative reactions of the uses of a composition such as for use in an enzyme-linked (immuno)assays, preferably in immunosorbent assay (ELISA) and for distinguishing a negative reaction from a false negative reaction in the ELISA, which comprises in a mixture:
(a) a chromogenic substrate which is oxidizable by a peroxide generated in the ELISA in a reaction mixture to which a stop solution is then added to stop the reaction to form a first color with an absorbance at a first wavelength; and
(b) a pH indicator dye which is colorless or has a color which is essentially not detectable by eye when the composition is applied to a reaction vessel for the ELISA but which produces a second color detectable by eye and which has an absorbance at a second wavelength when an acid or base stop solution is added to the composition in the reaction vessel
   assay to be colorimetrically distinguished from false negative reactions. The following illustrates several of the preferred embodiments of the present invention.

The present invention provides a method for detecting an analyte in a peroxidase-based enzyme-linked immunosorbent assay (ELISA) for distinguishing a negative reaction from a false negative reaction in the ELISA, which comprises (a) providing a composition comprising a mixture of a chromogenic substrate which is oxidizable by peroxide in a reaction for the ELISA catalyzed by the peroxidase of the ELISA to form a first color with an absorbance at a first wavelength, a pH indicator dye which forms a second color which is detectable by eye and has an absorbance at a second wavelength when a stop solution is added to the composition in the reaction vessel, and a peroxide; (b) adding an aliquot of the composition to each of the reaction vessels for the ELISA to form a reaction mixture; (c) incubating the reaction mixture for a time sufficient to oxidize the chromogenic substrate to the first color; (d) adding the stop solution to the reaction mixture to stop the reaction and to generate the second color; and (e) measuring spectrographically absorbances at the first and second wavelengths, wherein absorbance at the first wavelength indicates the detection of the analyte, absorbance at only the second wavelength indicates the negative reaction, and absence of absorbance at the first and second wavelengths indicates the false negative reaction or detecting the second color by eye wherein the second color indicates that the reaction mixture contains the composition and the stop solution.

The present invention further provides an enzyme immunoassay for distinguishing a negative reaction from a false negative reaction in the immuno-assay for colorimetric detection of an analyte of a type wherein a quantity of a first immunologic reagent such as an antibody, Fab fragment, Fv fragment, single-chain Fv polypeptide, or other antibody derivative is adsorbed to a solid support in a reaction vessel; a conjugate is formed between a second immunologic reagent and a peroxidase; the conjugate is admixed with a sample to be tested for the analyte, the analyte binds to the first immunologic reagent and to the conjugate to form an immunologic complex in solid phase; and the quantity of the analyte is determined by measuring the reaction of the immunologic complex with a chromogenic substrate oxidizable by peroxide and the peroxidase, wherein the improvement comprises (a) providing a liquid solution comprising the chromogenic substrate, peroxide, and a pH indicator dye, wherein the chromogenic substrate is oxidizable to a first color with an absorbance at a first wavelength and the pH indicator dye produces a second color which is detectable by eye and which has an absorbance at a second wavelength when a stop solution is added to the reaction vessel containing the liquid solution; (b) mixing the liquid solution with the immunologic complex in the reaction vessel to form a reaction mixture which oxidizes the chromogenic substrate; (c) adding the stop solution to the reaction mixture in the reaction vessel to produce the first and second colors; and, (d) measuring spectrographically the absorbances at the first and second wavelengths, wherein absorbance at the first wavelength indicates detection of the analyte, absorbance at the second wavelength indicates a negative reaction, and an absence of absorbance at the first or second wavelength indicates a false negative reaction or detecting the second color by eye wherein the second color indicates that the reaction mixture contains the liquid solution and the stop solution.

It is provided a enzyme immunoassay for distinguishing a negative reaction from a false negative reaction for colorimetric detection of an antibody of a type wherein a quantity of an analyte is adsorbed to a solid support in a reaction vessel; a conjugate is formed between an immunologic reagent such as an antibody, Fab fragment, Fv fragment, single-chain Fv polypeptide, or other antibody derivative and a peroxidase the conjugate is admixed with a sample to be tested for the antibody, the antibody binds to the analyte and to the conjugate to form an immunologic complex in solid phase; and the quantity of the antibody is determined by measuring the reaction of the immunologic complex with a chromogenic substrate oxidizable by peroxide and the peroxidase, wherein the improvement comprises (a) providing a liquid solution comprising the chromogenic substrate, peroxide, and a pH indicator dye, wherein the chromogenic substrate is oxidizable to a first color with an absorbance at a first wavelength and the pH indicator dye produces a second color which is detectable by eye and which has an absorbance at a second wavelength when a stop solution is added to the reaction vessel containing the liquid solution; (b) mixing the liquid solution with the immunologic complex in the reaction vessel to form a reaction mixture which oxidizes the chromogenic substrate; (c) adding the stop solution to the reaction mixture in the reaction vessel to produce the first and second colors; and, (d) measuring spectrographically the absorbances at the first and second wavelengths, wherein absorbance at the first wavelength indicates detection of the analyte, absorbance at the second wavelength indicates a negative reaction, and an absence of absorbance at the first or second wavelength indicates a false negative reaction or detecting the second color by eye wherein the second color indicates that the reaction mixture contains the liquid solution and the stop solution.

Preferably, in the above methods, the pH, indicator is colorless or has a color which is essentially not detectable by eye when the liquid solution is applied to the reaction vessel for the immunoassay but produces the second color which is detectable by eye and which has the absorbance at the second wavelength when the stop solution is added to the reaction vessel containing the liquid solution.

In a further embodiment of the above methods, the stop solution is an acid and the chromogenic substrate is selected from the group consisting of ortho-phenylenediamine (OPD), 2,2'-azinobis-(3-ethyl-benz-othiazoline-6-sulfonic acid) (ABTS), diaminobenzidine (DAB), 3,3'dimethyloxybenzidine (ortho-dianisidine or ODN), and 3,3',5,5'-tetramethylbenzidine (TMB). In a further embodiment, the stop solution is an acid and the chromogenic substrate is 2,2'-azinobis-(3-ethyl-benzothiazoline-6-sulfonic acid) (ABTS).

In a further still embodiment of the above methods, the pH indicator dye is selected from the group consisting of cresol red, m-cresol purple, benzopurpurin 4B, metanil yellow, 4-phenylazodiphenylamine, malachite green, orange IV, 2,2',2",4,4"-pentamethoxytriphenyl carbinol, and combinations thereof. In a preferred embodiment, the pH indicator dye is m-cresol purple.

In a further still embodiment of the above methods, the stop solution is a base and the chromogenic substrate is 5-aminosalicylic acid (5AS) and in a further still embodiment, the pH indicator dye is selected from the group consisting of phenolphthalein, thymolphthalein, alizarin Yellow R, indigo carmine, and combinations thereof.

In further embodiments of the above methods, the ELISA is a direct immunoassay, an indirect immunoassay, or a competitive immunoassay.

The present invention further provides a method for detecting an analyte using 3,3',5,5'-tetramethylbenzidine (TMB) as a chromogenic substrate in an enzyme-linked immunosorbent assay (ELISA) and distinguishing a negative reaction from a false negative reaction in the ELISA, which comprises (a) providing a composition comprising a mixture of the TMB which is oxidizable by peroxide in a reaction for the ELISA catalyzed by the peroxidase of the ELISA to form a first color with an absorbance at a first wavelength, a pH indicator dye which is colorless or has a color which is essentially not detectable by eye when the composition is applied to a reaction vessel for the ELISA but which forms a second color which is detectable by eye and has an absorbance at a second wavelength when a stop solution is added to the composition in the reaction vessel, and a peroxide; (b) adding an aliquot of the composition to each of the reaction vessels for the ELISA to form a reaction mixture; (c) incubating the reaction mixture for a time sufficient to oxidize the TMB to the first color; (d) adding the stop solution to the reaction mixture to stop the reaction, which produces the second color and further oxidizes the TMB to the first color; and (e) measuring spectrographically absorbances at the first and second wavelengths, wherein absorbance at the first wavelength indicates the detection of the analyte, absorbance at only the second wavelength indicates the negative reaction, and absence of absorbance at the first and second wavelengths indicates the false negative reaction or detecting the second color by eye wherein the second color indicates that the reaction mixture contains the composition and the stop solution.

The present invention further provides uses of an enzyme immunoassay for distinguishing a negative reaction from a false negative reaction for colorimetric detection of an analyte of a type wherein a quantity of a first antibody is adsorbed to a solid support in a reaction vessel; a conjugate is formed between an immunologic reagent and a peroxidase; the conjugate is admixed with a sample to be tested for the analyte, the analyte binds to the first antibody and to the conjugate to form an immunologic complex in solid phase; and the quantity of the analyte is determined by measuring the reaction of the immunologic complex with 3,3',5,5'-tetramethylbenzidine (TMB) which is oxidizable by peroxide and the peroxidase, wherein the improvement comprises (a) providing a liquid solution comprising the TMB, peroxide, and a pH indicator dye, wherein the TMB is oxidizable to a first color with an absorbance at a first wavelength and the pH indicator dye is colorless or has a color which is essentially not detectable by eye when the liquid solution is applied to the reaction vessel for the immunoassay but which produces a second color which is detectable by eye and which has an absorbance at a second wavelength when a stop solution is added to the reaction vessel containing the liquid solution; (b) mixing the liquid solution with the immunologic complex in the reaction vessel to form a reaction mixture which oxidizes the TMB; (c) adding the stop solution to the reaction mixture in the reaction vessel to produce the first and second colors; and, (d) measuring spectrographically the absorbances at the first and second wavelengths, wherein absorbance at the first wavelength indicates detection of the analyte, absorbance at the second wavelength indicates a negative reaction, and an absence of absorbance at the first,or second wavelength indicates a false negative reaction or detecting the second color by eye wherein the second color indicates that the reaction mixture contains the liquid solution and the stop solution.

The present invention further provides uses of an enzyme immunoassay for distinguishing a negative reaction from a false negative reaction for colorimetric detection of an antibody of a type wherein a quantity of an analyte is adsorbed to a solid support in a reaction vessel; a conjugate is formed between an immunologic reagent such as an antibody, Fab fragment, Fv fragment, single-chain Fv polypeptide, or other antibody derivative and a peroxidase; the conjugate is admixed with a sample to be tested for the antibody, the antibody binds to the analyte and to the conjugate to form an immunologic complex in solid phase; and the quantity of the antibody is determined by measuring the reaction of the immunologic complex with 3,3',5,5'-tetramethylbenzidine (TMB) which is oxidizable by peroxide and the peroxidase, wherein the improvement comprises (a) providing a liquid solution comprising the TMB, peroxide, and a pH indicator dye, wherein the TMB is oxidizable to a first color with an absorbance at a first wavelength and the pH indicator dye is colorless or has a color which is essentially not detectable by eye when the liquid solution is applied to the reaction vessel for the immunoassay but which produces a second color which is detectable by eye and which has an absorbance at a second wavelength when a stop solution is added to the reaction vessel containing the liquid solution; (b) mixing the liquid solution with the immunologic complex in the reaction vessel to form a reaction mixture which oxidizes the TMB; (c) adding the stop solution to the reaction mixture in the reaction vessel to produce the first and second colors; and, (d) measuring spectrographically the absorbances at the first and second wavelengths, wherein absorbance at the first wavelength indicates detection of the analyte, absorbance at the second wavelength indicates a negative reaction, and an absence of absorbance at the first or second wavelength indicates a false negative reaction or detecting the second color by eye wherein the second color indicates that the reaction mixture contains the liquid solution and the stop solution.

In a further embodiment of the method, the pH indicator dye is selected from the group consisting of cresol red, m-cresol purple, benzopurpurin 4B, metanil yellow, 4-phenylazodiphenylamine, malachite green, orange IV, 2,2',2",4,4" -pentamethoxytriphenyl carbinol, and combinations thereof. In a preferred embodiment, the pH indicator dye is m-cresol purple. In a further still embodiment, the first wavelength is about 450 nm and the second wavelength is over 500 nm, and further still the acid is selected from the group consisting of HCL, H₂SO₄, and H₂PO₄.

In further embodiments of the method, the ELISA is a direct immunoassay, an indirect immunoassay, or a competitive immunoassay.

The present invention further provides the use of a kit for detecting an analyte in an enzyme-linked assay and distinguishing a negative reaction from a false negative reaction in the assay, which comprises one or more first containers each of which contains a mixture of a chromogenic substrate, a pH indicator dye suitable for use with the chromogenic substrate, which is colorless or has a color which is essentially not detectable by eye when the mixture is applied to a reaction vessel for the enzyme-linked assay but which produces a color detectable by eye when an acid or base stop solution is added to the mixture in the reaction vessel, and a peroxide.

The present invention further provides the use of a kit for an enzyme-linked immunosorbent assay (ELISA) for detecting an analyte and distinguishing a negative reaction from a false negative reaction in the ELISA, which comprises one or more first containers each of which contains a mixture of a chromogenic substrate, a pH indicator dye suitable for use with the chromogenic substrate, which is colorless or has a color which is essentially not detectable by eye when the mixture is applied to a reaction vessel for the ELISA but which produces a color detectable by eye when an acid or base stop solution is added to the mixture, and a peroxide.

In a further embodiment of the above kits, the first container contains a mixture of a chromogenic substrate and a pH indicator dye and the peroxide is contained in a second container. In a further still embodiment, the kit further includes a third container containing an acid or base stop.

In a further still embodiments of the above kits, the chromogenic substrate is selected from the group consisting of ortho-phenylenediamine (OPD), 2,2'-azinobis-(3-ethyl-benzothiazoline-6-sulfonic acid) (ABTS), diaminobenzidine (DAB), 3,3'dimethyloxybenzidine (ortho-dianisidine or ODN), and 3,3',5,5'-tetramethylbenzidine (TMB).

In a further still embodiments of the above kits, the pH indicator dye is selected from the group consisting of cresol red, m-cresol purple, benzopurpurin 4B, metanil yellow, 4-phenylazodiphenylamine, malachite green, quinaldine red, orange IV, 2,2',2",4,4"-pentamethoxytriphenyl carbinol, and combinations thereof.

In a further still embodiments of the above kits, the chromogenic substrate is 5-aminosalicylic acid (5AS) and further still, the pH indicator dye is selected from the group consisting of phenolphthalein, thymolphthalein, alizarin yellow R, indigo carmine, and combinations thereof.

In a further embodiments of the above kits, the pH indicator dye is m-cresol purple and the chromogenic substrate is selected from the group consisting of 3,3',5,5'-tetramethylbenzidine (TMB) and 2,2'-azinobis-(3-ethyl-benzothiazoline-6-sulfonic acid) (ABTS).

The present invention further provides the use of a kit for an enzyme-linked immunosorbent assay (ELISA) for detecting an analyte for distinguisting a negative reaction from a false negative reaction in the assay, which comprises one or more first containers each of which contains a mixture of 3, 3' , 5, 5' -tetramethylbenzidine (TMB), a pH indacator dye suitable for use with the chromogenic substrate, which is colorless or has a color which is essentially not detectable by eye when the mixture is applied to a reaction vessel for the ELISA but which produces a color detectable by eye when an acid or base stop solution is added to the mixture in the reaction vessel, and a peroxide. Preferably, the pH indicator is m-cresol purple.

In a further embodiment of the kit, the first container contains a mixture of the TMB and a pH indicator dye and the peroxide is contained in a second container. In a further embodiment, the kit further includes a third container containing an acid or base stop.

The present invention further provides a composition for use, in an enzyme-linked immunosorbent assay (ELISA) for distinguishing a negative reaction to from a false negative reaction in the ELISA, which comprises in a mixture (a) a chromogenic substrate which is oxidizable by a peroxide generated in the ELISA in a reaction mixture to which a stop solution is then added to stop the reaction to form a first color; and (b) a pH indicator dye which is colorless or has a color which is essentially not detectable by eye when the composition is applied to a reaction vessel for the ELISA but which produces a second color detectable by eye and which has an absorbance at a second wavelength when an acid or base stop solution is added to the composition in the reaction vessel.

In a further embodiment of the composition, the chromogenic substrate is selected from the group consisting of ortho-phenylenediamine (OPD), 2,2'-azinobis-(3-ethyl-benzothiazoline-6-sulfonic acid) (ABTS), diaminobenzidine (DAB), and 3,3'dimethyloxybenzidine (ortho-dianisidine or ODN).

In a further still embodiment of the composition, the pH indicator dye is selected from the group consisting of cresol red, m-cresol purple, benzopurpurin 4B, metanil yellow, 4-phenylazodiphenylamine, malachite green, quinaldine red, orange IV, 2,2',2",4,4"-pentamethoxytriphenyl carbinol, and combinations thereof.

In a further still embodiment of the composition, the chromogenic substrate is 5-aminosalicylic acid (5AS) and further still, the pH indicator dye is selected from the group consisting of phenolphthalein, thymolphthalein, alizarin yellow R, indigo carmine, and combinations thereof.

In a further still embodiment of the composition, the pH indicator dye is m-cresol purple and the chromogenic substrate is 2,2'-azinobis-(3-ethyl-benzothiazoline-6-sulfonic acid) (ABTS).

The present invention further provides a composition for use in an enzyme-linked immunosorbent assay (ELISA) and for distinguishing a negative reaction from a false negative reaction in the ELISA, which comprises in an aqueous mixture (a) 3, 3', 5, 5'-tetramethylbenzidine (TMB) which is oxidizable by a peroxide generated in the ELISA in a reaction mixture to which a stop solution is then added to stop the reaction to form a first color; and (b) an indicator dye which is colorless or has a color which is essentially not detectable by eye when the composition is applied to a reaction vessel for the ELISA but which produces a color detectable by eye and which has an absorbance at a second wavelength when an acid or base stop solution is added to the composition in the reaction vessel.

In a further embodiment of the composition, the pH indicator dye is selected from the group consisting of cresol red, benzopurpurin 4B, metanil yellow, 4-phenylazodiphenylamine, malachite green, quinaldine red, orange IV, 2,2',2",4,4"-pentamethoxytriphenyl carbinol, and combinations thereof.

In a further still embodiment of the composition, the pH indicator dye is m-cresol purple.

In a further embodiment, the present invention provides a method for detecting an analyte in a enzyme-linked assay and distinguishing a negative reaction from a false negative reaction in the assay, which comprises (a) providing a composition comprising a mixture of a chromogenic substrate selected from the group consisting of ortho-phenylenediamine (OPD), 2,2'-azinobis-(3-ethyl-benzothiazoline-6-sulfonic acid) (ABTS), diaminobenzidine (DAB), and 3,3'dimethyloxybenzidine (ortho-dianisidine or ODN) which is converted by an enzyme in a reaction vessel for the assay to a first color which has an absorbance at a first wavelength and a pH indicator dye which produces a second color detectable by eye and which has an absorbance at a second wavelength when an acid or base stop solution is added to the composition in the reaction vessel; (b) adding an aliquot of the composition to the reaction vessels for the enzyme-linked assay to form a reaction mixture; (c) incubating the reaction mixture for a time sufficient to produce the first color; (d) adding the stop solution to the reaction mixture to stop the reaction and to generate the second color; and (e) measuring spectrographically absorbances at the first and second wavelengths, wherein absorbance at the first wavelength indicates the detection of the analyte, absorbance at only the second wavelength indicates the negative reaction, and absence of absorbance at the first and second wavelengths indicates the false negative reaction or detecting the second color by eye wherein the second color indicates that the reaction mixture contains the composition and the stop solution.

In a further embodiment of the method, the enzyme is an alkaline phosphatase, the chromogenic substrate is p-nitrophenyl phosphate, and the stop solution is a base. In a further still embodiment, the pH indicator dye is selected from the group consisting of alizarin Yellow R, indigo carmine, cresol red, m-cresol purple, benzopurpurin 4B, metanil yellow, 4-phenylazodiphenylamine, malachite green, orange IV, 2,2',2'',4,4''-pentamethoxytriphenyl carbinol, and combinations thereof.

In further still embodiments, the assay is a direct immunoassay, an indirect immunoassay, or a competitive immunoassay. In a further embodiment, the assay is an enzyme-linked immunosorbent assay (ELISA).

The present invention further provides a use of a kilt for detecting an analyte in an enzyme-linked assay and distinguishing a negative reaction from a false negative reaction in the assay, which comprises one or more containers each of which contains a mixture of a chromogenic substrate selected from the group consisting of ortho-phenylenediamine (OPD), 2,2'-azinobis-(3-ethyl-benzothiazoline-6-sulfonic acid) (ABTS), diaminobenzidine (DAB), and 3,3'dimethyloxybenzidine (ortho-dianisidine or ODN) which is converted by an enzyme in a reaction vessel for the assay to a first color and a pH indicator dye suitable for use with the chromogenic substrate which produces a second color detectable by eye when an acid or base stop solution is added to the mixture in the reaction vessel..

In a further embodiment of the kit, the chromogenic substrate is p-nitrophenyl phosphate. In a further still embodiment, the pH indicator dye is selected from the group consisting of alizarin Yellow R, indigo carmine, cresol red, m-cresol purple, benzopurpurin 4B, metanil yellow, 4-phenylazodiphenylamine, malachite green, orange IV, 2,2',2",4,4"-pentamethoxytriphenyl carbinol, and combinations thereof.

In a further embodiment of the kit, the assay is an enzyme-linked immunosorbent assay (ELISA).

The present invention further provides a composition for use in an enzyme-linked assay and which enables a negative reaction to be distinguished from a false negative reaction in the assay, which comprises in a mixture (a) a chromogenic substrate selected from the group consisting of ortho-phenylenediamine (OPD), 2,2'-azinobis-(3-ethyl-benzothiazoline-6-sulfonic acid) (ABTS), diaminobenzidine (DAB), and 3,3'dimethyloxybenzidine (ortho-dianisidine or ODN) which is converted by an enzyme in a reaction vessel for the assay to a first color; and (b) a pH indicator dye which is colorless or has a color which is essentially not detectable by eye when the composition is applied to a reaction vessel for the ELISA but which produces a second color detectable by eye and which has an absorbance at a second wavelength when an acid or base stop solution is added to the composition in the reaction vessel.

In a further embodiment of the composition, the chromogenic substrate is p-nitrophenyl phosphate. In a further still embodiment, the pH indicator dye is selected from the group consisting of alizarin Yellow R, indigo carmine, cresol red, m-cresol purple, benzopurpurin 4B, metanil yellow, 4-phenylazodiphenylamine, malachite green, orange IV, 2,2',2",4,4"-pentamethoxytriphenyl carbinol, and combinations thereof.

In a further embodiment of the composition, the assay is an enzyme-linked immunosorbent assay (ELISA).

### OBJECTS

It is an object of the present invention to provide a method for distinguishing negatives from false negatives in assays which does not interfere with positive reactions.

It is a further object of the present invention to provide a method for distinguishing negatives from false negatives in peroxide-linked assays which does not interfere with positive reactions.

It is a further object of the present invention to provide a method for distinguishing negatives from false negatives in ELISAs which does not interfere with positive reactions.

It is a further object of the present invention to provide a method for distinguishing negatives from false negatives in peroxide-linked ELISAs which does not interfere with positive reactions.

These and other objects of the present invention will become increasingly apparent with reference to the following drawings and preferred

### DESCRIPTION OF THE DRAWINGS

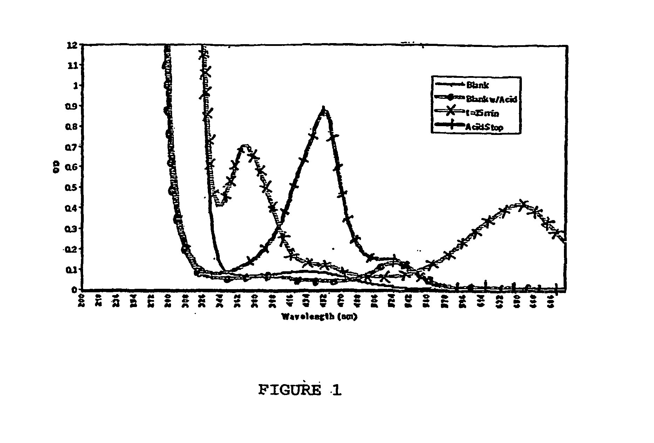

Figure 1 shows comparative absorbance spectra for the composition comprising TMB and m-cresol purple with and without horseradish peroxidase (HRP). The black line (-) is the absorbance spectrum for a sample containing the composition. The green line (-●-) shows the absorbance spectrum for a sample containing the composition and acid stop. The blue line (-x-) shows the absorbance spectrum for a sample containing the composition incubated with HRP for 15 minutes. The red line (-|-) shows the absorbance spectrum for a sample containing the composition incubated with HRP for 15 minutes followed by adding the'acid stop. All spectra were measured against water on a Shimadzu UV-1601 spectrophotometer.

### DETAILED DESCRIPTION OF THE INVENTION

The term "chromogenic substrate" includes chemicals which can participate in particular enzymatic reactions as either donors or acceptors for the reaction and which changes color during the reaction. For example, peroxidase converts hydrogen peroxide to water. It does so by obtaining two hydrogens from a donor molecule. When the donor molecule is a chromogenic substrate, the oxidation of the chromogenic substrate causes the substrate to change to a detectable color. For example, 3,3',5,5'-tetramethylbenzidine (TMB) is colorless in the reduced state but blue in the oxidized state or yellow in the diamine state. In the case of alkaline phosphatase reactions, the chromogenic substrate is converted from a colourless form to a visible color. For example, when the substrate is p-nitrophenyl phosphate (pNPP), the alkaline phosphatase converts it to p-nitrophenoxide (benzenoid form) which is colorless. However, at alkaline pH, the benezoid form is converted to a quinonoid form which is yellow.

The term "pH indicator" as used herein includes chemicals that take on different colors depending upon the concentration of hydronium or hydroxide ion present in an aqueous solution. Many pH indicators change from one color to another or from colorless to a color over a particular pH range. This is called the pH indicator range and this range varies from pH indicator to pH indicator. The term "pH indicator dye" as used herein further includes any dye which changes color or produces a color upon a change in pH but which is not commonly used as an indicator for detecting pH changes *per se.*

The phrase "has a color which is essentially not detectable by eye when applied to a reaction vessel for an assay" means that a composition comprising the pH indicator dye and chromogenic substrate has a color which an operator of the assay would not readily perceive in the normal course of performing the assay. A color which is not readily perceptible is a color which is too faint or of insufficient intensity to be detected by the unaided human eye, particularly at the volume which is applied to the reaction vessel and particularly when the composition is at a pH between about 3 and 6.5. In general, as the volume of a solution with a detectable color decreases, the less detectable or perceptible to the eye becomes the color of the solution. For example, while the chromogenic substrate and pH indicator dye compositions of the present invention might have a color which in a large volume might be perceptible or detectable by eye, the volume applied to a reaction vessel for an assay is not sufficient to enable the color to be readily perceptible or detectable by the operator's eye.

The phrase "normal course of performing a reaction in an assay" means performing an assay using a volume of the chromogenic substrate and pH indicator dye which is routinely used for the type of assay. For example, many ELISAs use between about 50 to 500 µL of chromogenic substrate and peroxide solution per reaction well. Thus, in the present invention, the well would contain about 50 to 500 µL of the chromogenic substrate, pH indicator dye, and peroxide solution per reaction well.

The term "ELISA" refers to enzyme-linked immunosorbent assay and includes direct, indirect, and competitive ELISAs. The basic principle of an ELISA is to use an enzyme to detect the binding of antigen and antibody. The enzyme converts a colorless chromogenic substrate to a colored product, indicating the presence of antigen-antibody binding. An ELISA can be used to detect either the presence of particular antibodies or antigens in a sample. A direct ELISA is used primarily to determine the amount of antigen in a sample. In a direct ELISA, antibody for a particular antigen is immobilized in the reaction vessel or well of a microtiter plate or other solid support. The immobilized antibody binds the antigen in a sample and the bound antigen is detected by an enzyme-labeled antibody specific for the antigen. An indirect ELISA is used primarily to determine the strength or amount of an antibody response to a particular antigen in a sample such as serum from an animal. In an indirect ELISA, an antigen is immobilized in the reaction vessel or well of a microtiter plate or other solid support. The immobilized antigen binds antibody in a sample which is specific for the antigen and the bound antibody is detected by an enzyme-labeled antibody specific for the bound antibody. For both direct and indirect ELISAs, the amount of colored product is directly proportional to the amount of antigen or antibody in the sample. A competitive ELISA is a variation where either (a) the amount of antibody in a sample is determined in a competition reaction between antibody in the sample and enzyme-labeled antibody for an antigen immobilized in the reaction vessel or well of a microtiter plate or other solid support or (b) the amount of antigen in a sample is determined in a competition reaction between the antigen in the sample and enzyme-labeled antigen for antibody specific for the antigen immobilized in the reaction vessel or well of a microtiter plate or other solid support. In a competitive ELISA, the amount of colored product is inversely proportional to the amount of antibody or antigen in the sample.

The term "immunoassay" includes all methods for detecting an antigen or antibody in a sample such as ELISAs and the like, and includes direct, indirect, and competitive immunoassays.

The term "analyte" includes both antigen (including haptens and lectins) and antibody. Whether the analyte is an antigen or an antibody depends on the type of immunoassay. For example, in immunoassays for detecting an antigen, the antigen is the analyte, whereas in immunoassays for detecting an antibody, the antibody is the analyte.

The terms "immunologic reagent" and "antibody" include whole antibodies and derivatives thereof. For example, the terms include Fab fragments, recombinant Fab polypeptides, Fv fragments, recombinant single-chain Fv polypeptides, and variations thereof. The terms also include both polyclonal antibodies and monoclonal antibodies and antibodies and derivatives thereof made by recombinant DNA methods.

The term "reaction vessel" includes test tube, well of a microtiter or tissue culture plate, capillary tube, cuvette, and the like.

The present invention provides a solution to the problem of whether a negative reaction in enzyme-linked assays such as ELISAs (direct, indirect, and competitive), immunoassays (direct, indirect, and competitive), hybridizations, or the like, is negative because the test sample did not contain the analyte being tested for or is negative because one or more reagents necessary for the reaction had been omitted by including in a ready-to-use composition for enzyme-linked assays a pH indicator dye which produces a detectable color or absorbance when a stop solution is added to the composition in the reaction vessel. In a preferred embodiment, the pH indicator dye is colorless or has a color which is not detectable by eye when the composition is applied to a reaction vessel for the assay but which produces the detectable color when the stop solution is added to the composition in the reaction vessel. The present invention is particularly useful for peroxidase-linked assays, particularly immunoassays such as ELISAs, and particularly is those assays which use an acid or base stop step to enhance sensitivity of the assay. In a preferred embodiment, the peroxidase is horseradish peroxidase (HRP). The present invention is also useful for alkaline phosphatase-linked assays, particularly in ELISAs which use p-nitrophenyl phosphate as the substrate.

In a typical peroxidase-linked immunoassay, the peroxidase is conjugated to a first member of a binding pair, for example an antibody or analyte. The peroxidase-binding pair conjugate is incubated with a sample which is suspected of containing the second member of the binding pair (analyte or antibody) in a reaction vessel. After a time sufficient to enable the peroxidase-binding pair conjugate to bind the second member of the binding pair to form a peroxidase-labeled complex, unbound material is removed and the peroxidase-labeled complex is detected by incubating the complex in a solution comprising a peroxide and a chromogenic substrate which is preferably in a water-soluble leuco form- The peroxidase catalyzes oxidation by the peroxide of the chromogenic substrate to a detectable color. For particular chromogenic substrates such as 3,3',5,5'-tetramethylbenzidine (TMB) or orthophenylenediamine (OPD), the sensitivity of the reaction is enhanced by stopping the oxidation reaction with a stop solution containing an acid, which in the case of TMB is an increase of sensitivity of about 2 to 4 fold. A preferred peroxidase is HRP. HRP is most active between about pH 5 and pH 7.

In the present invention, the pH indicator dye is preferably selected to be colorless or have a color which is essentially undetectable by eye when a composition comprising a chromogenic substrate and the pH indicator dye is applied to a reaction vessel for the assay (the concentration of the pH indicator dye in the composition, the pH of the composition, and the volume of the composition together results in the color of the pH indicator dye being essentially undetectable by eye) but to produce a color which is detectable by eye and by absorbance when the pH of the composition is lowered to below the reaction pH by addition of an acid stop solution to the reaction vessel or raised to a pH above the reaction pH by addition of a base stop solution to the reaction vessel. However, in particular embodiments comprising particular chromogenic substrates, the pH indicator can have a color which is detectable by eye when a composition comprising a chromogenic substrate and the pH indicator dye is applied to a reaction vessel for the assay. Upon addition of the stop solution, the color can remain the same or the color can be changed to another color.

An additional requirement for the pH indicator dye is that the color or absorbance wavelength which is produced upon addition of the acid or base does not substantially interfere with or otherwise substantially affect detection of the absorbance wavelength produced by oxidation of the chromogenic substrate in positive reactions. In other words, the color produced by the pH indicator dye has an absorbance range which is outside of or does not substantially interfere with the absorbance range for the chromogenic substrate after acid or base has been added to the reaction. Preferably, the pH indicator dye is transparent in the absorbance range of the chromogenic substrate color produced prior to the addition of the acid or base. For example, TMB is oxidized to a blue color which is converted to a yellow upon addition of acid.

A further requirement for the pH indicator dye is that the pH indicator dye must be substantially non-interfering in the reaction between the chromogenic substrate and the enzyme.

In a particularly useful embodiment of the present invention, ready-to-use compositions are provided which comprise a mixture of the pH indicator dye, a chromogenic substrate, and a peroxide. Preferably, the composition further includes a buffer and other compounds which might stabilize the chromogenic substrate and/or pH indicator prior to or during the reaction or facilitate solubility of the chromogenic substrate in the reaction or during storage when the composition is provided as an aqueous solution. Most preferably, the compositions are liquid.

Suitable chromogenic substrates for peroxidase-linked assays include, but are not limited to, ortho-phenylenediamine (OPD), 2,2'-azinobis-(3-ethyl-benzothiazoline-6-sulfonic acid) (ABTS), diaminobenzidine (DAB), 3,3'dimethyloxybenzidine (orthodianisidine or ODN), 5-aminosalicylic acid (5AS), and 3,3',5,5'-tetraalkylbenzidine such as 3,3',5,5'-tetramethylbenzidine (TMB). Other chromogenic substrates include various fluorescent and chemiluminescent compounds.

OPD is colorless but is oxidized in a peroxidase reaction to a yellow color with a maximum absorbance at 450 nm. The addiction of acid to the reaction produces a stable orange color with an absorbance maximum at 490 nm. ABTS is oxidized in a peroxidase reaction to a blue-green color with a maximum absorbance at 405-410 nm. The addition of 1% SDS or acid to the reaction produces a stable blue-green color with an absorbance maximum at 405-410 nm. DAB is oxidized to an insoluble brown precipitate. As such, it is used primarily for immunohistochemical assays. OND is oxidized in a peroxidase reaction to a yellow-orange color with an absorbance maximum at 460 nm. The reaction can be stopped by the addition of an acid such as HCL and the reaction product read. 5AS is oxidized in a peroxidase reaction to a brown color with an absorbance maximum at 450 nm. The reaction can be stopped by the addition of NaOH and the reaction product read at 550 nm. TMB is colorless but is oxidized in a peroxidase reaction to a blue color with a maximum absorbance at 650 nm. The addition of acid to the reaction produces a stable yellow color with an absorbance maximum at 450 nm. Of the above, TMB is most preferred by those skilled in the art because it is non-hazardous and the most widely used of chromogenic substrates for peroxide-linked immunoassays such as ELISAs and the like. The present invention includes compositions comprising any one of the above chromogenic substrates in combination with a pH indicator dye and peroxide.

It is preferred that the chromogenic substrate be provided in an aqueous or water-base solution. Preferably, the aqueous solution is 100% water-based and does not include any organic solvent. It is further preferable that the solution be weakly buffered. In particular compositions, it may be necessary or desirable to include one or more solubility increasing agent(s) in order to facilitate the (permanent) dissolution of the chromogenic substrate. An example of a suitable solubility increasing agent is polyvinyl alcohol. Thus, in particular embodiments, the chromogenic substrate can comprise a solvent system comprising less than 5% (v/v) of organic solvents. It is further preferable that the chromogenic substrate be provided in mixture with a peroxide such as hydrogen peroxide or urea peroxide.

In a preferred embodiment, the chromogenic composition comprises TMB in a solution which is preferably aqueous or water-based. Examples of commercially available TMB chromogenic compositions which can be modified to include a pH indicator dye include, but are not limited to, K-BLUE SUBSTRATE, K-BLUE MAX SUBSTRATE, and the like available from Neogen Corp. Lansing, Michigan; TMB ONE, TMB PLUS, and the like available from Kem-En-Tec A/S, Copenhagen, Denmark; TMB substrate, TMB substrate with tracking dye, and the like available from BioFX Laboratories, Inc., Owning Mills, Maryland; and, TMB Subtrate Kit available from Vector Laboratories, Inc. Burlingame, California. In a most preferred embodiment, the TMB chromogenic composition comprises a 100% aqueous or water-based solution which is the same as or similar to the TMB chromogenic solution disclosed in U.S. Patent No. 5,206,150 to Tai. In a particularly preferred embodiment, the TMB chromogenic composition does not contain any organic solvents. It is further preferable that the solution be weakly buffered.

The pH indicator dye which is preferred for the present invention is colorless at a pH greater than about 2.8 or provides a color at a pH greater than about 2.8 but less than about pH 7.0 that is essentially not detectable by eye at the concentration or volume used in the reaction of an assay. Preferably, the pH indicator dye is water-soluble in at least the oxidized state or the reduced state. Preferably, the pH indicator dye is water-soluble in both states. It is particularly important that the pH indicator dye change color at a pH which is below or above the pH in which the chromogenic substrate oxidation or reduction reaction is performed and that the color, which is produced by the pH indicator dye when the reaction is stopped by addition of acid or base, is readily detectable by eye but substantially transparent in the absorbance range of the oxidized or reduced chromogenic substrate. However, it is preferable that the pH indicator also have an absorbance at a wavelength which does not interfere with the absorbance range of the oxidized or reduced chromogenic substrate. Thus, the color produced by the pH indicator dye is not only visible to the eye but is measurable at a wavelength other than the wavelength used to measure the oxidized or reduced chromogenic substrate. This is particularly useful for analyses wherein a detector is adapted to measure the absorbance of the oxidized or reduced chromogenic substrate and the absorbance of the oxidized or reduced pH indicator. Such detectors include ELISA readers.

Examples of pH indicator dyes which change color at an acid pH and are useful in the present invention include, but are not limited to, cresol red (yellow to red transition at pH 0.5-1.8), m-cresol purple (yellow to red transition at pH 1.2-2.8), 2,2',2",4,4'-pentamethoxytriphenyl carbinol (colorless to red transition at pH 1.2 to 3.2), and benzopurpurin 4B (red to violet transition at pH 2.2-4.2). Other acid pH indicator dyes which might be useful include but are not limited to, metanil yellow (yellow to red transition at pH 1.2-3.0), 4-phenylazodiphenylamine (yellow to red transition at pH 1.2-2.5), malachite green (yellow to blue-green transition at pH 0.2-1.8), quinaldine red (colorless to red transition at pH 1.0-2.2), orange IV (yellow to red transition at pH 1.4-2.8), thymol blue (red to yellow transition at pH 1.2 to 2.8), xylenol blue (yellow to red transition at pH 1.8 to 2.8), and combinations thereof.

Examples of pH indicator dyes which change color at an alkaline pH and are useful with chromogenic substrates such as 5AS include, but are not limited to, such dyes as phenolphthalein (colorless to red transition at pH 8.0 to 10), thymolphthalein (colorless to blue transition at pH 8.8 to 10.5), alizarin Yellow R (yellow to violet transition at pH 10 to 12), indigo carmine (blue to yellow transition at pH 11.4 to 13), m-cresol purple (yellow to purple transition at pH 7.4 to 9.0), cresol red (yellow or orange to purple transition at pH 7.0-8.8), thymol blue (yellow to blue transition at pH 8.0 to 9.6), xylenol blue (yellow to blue transition at pH 8.0 to 9.6), and combinations thereof.

The particular pH indicator dye used in an assay is that pH indicator dye which is suitable for use with the chromogenic substrate used in the assay. In other words, the pH indicator has an absorbance which does not interfere with the absorbance of the chromogenic substrate following the reaction and produces a color which is detectable by eye (visibly colored) after stopping the reaction with a stop solution but which is colorless or has a color which is essentially undetectable by eye when in the reaction vessel prior to adding the stop solution to the reaction. A preferred pH indicator dye for use in combination with TMB is m-cresol purple. The m-cresol purple is preferably used at a concentration which renders it essentially undetectable to the eye at the volume the TMB chromogenic solution is applied to a reaction vessel and at the pH of most TMB, chromogenic solutions (most TMB chromogenic solutions are weakly buffered, usually in a buffer between about pH 3.8 and about pH 6.5) but which still enables it to be detectable by eye when the pH of the solution is reduced by addition of a acid stop solution. The ability to detect by eye the m-cresol purple in a composition containing the TMB in a reaction vessel prior to adding the acid stop solution is a function of the concentration of the m-cresol purple in the composition and the volume of the composition in the reaction vessel. Thus, the m-cresol purple is at a concentration in the composition which renders it visually undetectable when the composition is applied to a reaction vessel.

When an acid stop solution is added to the composition in the reaction vessel, the pH is reduced to below pH 2.8 and the m-cresol purple is oxidized to a rose-pink color. The rose-pink color is detectable by eye even when the m-cresol purple is at a concentration and the composition is at a volume which renders it essentially undetectable by eye prior to adding the acid stop solution. The oxidized m-cresol purple has a maximum absorbance at about 524-542 nm. The maximum absorbance does not interfere with the maximum absorbance of TMB (or OPD, ODN, or ABTS). Thus, either by visual observation or by measuring the absorbance at 524-542 nm, a user of the composition can readily determine whether a negative reaction was because the sample did not contain the analyte or the composition had been omitted from the reaction.

In a typical detection reaction for a peroxidase conjugate (for example, an HRP conjugate), the reaction vessel comprises in aqueous mixture the peroxidase conjugate and a composition comprising an oxidizable chromogenic substrate such as TMB, OPD, OND, 5AS, ABTS, or the like, and a pH indicator dye with an absorbance at an acid pH or basic pH different from the absorbance of the oxidized chromogenic substrate at the acid pH or basic pH. For example, oxidized TMB, OPD, and ABTS at an acid pH have a maximum absorbance at 450, 490, and 405 nm, respectively, and acid-oxidized m-cresol purple has a maximum absorbance at 530 nm. Preferably, the above composition further includes hydrogen or urea peroxide. It is further preferable, that the ratio of the chromogenic substrate to the pH indicator dye is between 10 to 1 and 1 to 10.

During the reaction, the chromogenic substrate is oxidized or reduced to a state which has a first color or maximum absorbance wavelength. The reaction is then stopped by the addition of a stop solution comprising an acid (hydrochloric acid (HCL), sulfuric acid (H₂SO₄), phosphoric acid (H₂PO₄), or the like) or a base such as sodium hydroxide (NaOH). The acid or base stops the above oxidation or reduction of the chromogenic substrate and for some substrates such as TMB or OPD, causes a further oxidation of the chromogenic substrate to a second color or maximum absorbance wavelength. It also causes oxidation or reduction of the pH indicator dye to a third color or maximum absorbance wavelength. For other chromogenic substrates such as ABTS or OND, the color of the oxidized or reduced product does not change upon addition of the acid. For 5AS, the reaction is stopped with a base such as NaOH which converts the oxidized chromogenic substrate to a product with an absorbance maximum at 550 nm. For particular formulations of TMB, the color of the TMB remains blue after the addition of the acid stop.

In a typical reaction, a positive reaction containing peroxidase conjugate is detected visually by eye or by absorbance at the maximum absorbance wavelength for the oxidized chromogenic substrate and a negative reaction which does not contain peroxidase conjugate is detected visually by eye or by absorbance at the maximum absorbance wavelength for the oxidized pH indicator. In the case of false negatives-reactions such as those which contain peroxidase conjugate but not the composition comprising the chromogenic substrate, the acid, or both, the reaction is colorless by eye and has no absorbance at either the first or second or third maximum absorbance wavelengths. Thus, reactions which have no absorbance at the first or second maximum absorbance wavelengths but have absorbance at the third maximum absorbance wavelength are scored as negatives whereas all reactions with no absorbance at the third maximum absorbance wavelength are scored as false negatives.

In an automated ELISA reader, the ELISA reader will correctly score reactions with a first (without acid) or second maximum absorbance wavelength (with acid) as positives and reactions with the third maximum absorbance wavelength as negatives. In the case of a reaction in which either the acid (or base) or the composition had been omitted, there is no first, second, or third maximum absorbance wavelength. An automated ELISA reader will correctly score the above reaction as a false negative. Thus, the composition of the present invention not only enables detection of positive reactions but also enables negative reactions to be distinguished from false negatives.

While the above compositions comprise a pH indicator dye which is colorless or have a color which is essentially not detectable by eye when the composition is applied to a reaction vessel for the assay, in the case of assays comprising OPD, OND, 5AS, ABTS and some assays comprising TMB, the pH indicator dye comprising the composition can have a color which is detectable by eye when the composition is applied to the reaction vessel. The pH indicator dye does not need to be colorless or have a color which is essentially not detectable by eye when the composition is applied to the reaction vessel. However, the pH indicator dye comprising the composition preferably forms a second color which is detectable by eye and has an absorbance at a second wavelength when a stop solution is added to the composition in the reaction vessel. The second wavelength cannot substantially interfere with detection of the first wavelength for the color of formed by the chromogenic substrate.

In a preferred detection reaction, the reaction comprises HRP conjugate in an aqueous composition comprising TMB as the chromogenic substrate and a pH indicator dye such as m-cresol purple. Preferably, the above composition further includes hydrogen or urea peroxide. It is further preferable that the composition is 100% water-based. For many formulations, the ratio of TMB to pH indicator dye is usually between about 10 to 1 and 1 to 10. In general, the chromogenic solution and reaction have a pH of about 3.8 to 6.5. When the pH indicator dye is m-cresol purple, the preferred amount of the m-cresol purple is less than about 10 µg m-cresol purple sodium salt per mL of TMB substrate, more preferably, less than about 2.2 µg/mL, more preferable still about 1.1 µg/mL. This amount of m-cresol purple in solution with the TMB substrate renders it essentially indistinguishable or undetectable by eye at the volume normally used for a reaction in an assay from solutions without the m-cresol purple prior to the addition of stop solution at the volume but still produces a distinguishable or detectable color after addition of the stop solution.

During the reaction, the TMB is oxidized to a state which is visually blue and which has an absorbance maximum at about 650 nm. Upon addition of a stop solution (preferably, a stop solution which comprises HCL at about 1 M or H₂SO₄ at about 0.2 M), the pH of the reaction is reduced. The reduced pH stops the reaction and further oxidizes TMB in the blue state to a diamine state. In the diamine state, the oxidized TMB is visually yellow and has a maximum absorbance at about 450nm. Meanwhile, the m-cresol purple, which is essentially undetectable at pH 3.8 to 6.5 because of its low concentration in the composition (less than about 10 µg/mL) and the small reaction volume (in general, usually less than about 200 µL), is oxidized by the acid to a rose-pink (light red) which is visible to the eye and has a maximum absorbance at about 530-540 nm. Therefore, in a typical reaction, a positive reaction containing HRP conjugate can be detected visually by eye as a rose-pink or by absorbance at 450 nm and a negative reaction not containing HRP conjugate can be detected visually by eye as a rose-pink color or by measuring absorbance at about 520 to 565 nm, preferably at about 530 or 540 nm. In the case of false negatives-reactions such as those which contain HRP conjugate but in which the preferred composition, the acid stop, or both had been omitted, the reaction is colorless by eye and has no significant absorbance at 450 or 530 or 540 nm. Thus, reactions which have no absorbance at 450 nm but have absorbance at 530 or 540 nm are scored as negatives whereas all reactions with no absorbance at 530 or 540 nm are scored as false negatives.

In an automated ELISA reader, the ELISA reader will correctly score the yellow reactions as positives and the rose pink reactions as negatives. In the case of a reaction in which either the acid stop or the composition had been omitted, the color that is produced is blue or colorless, respectively. An automated ELISA reader will correctly score the above reactions as false negatives.

In a further embodiment of the above method using TMB as the chromogenic substrate, acid stops which stop the reaction but does not change the blue color of the reaction to yellow such as RED STOP (available from Neogen Corp., Lansing, MI) can be used. Thus, the absorbance of positive reactions remains blue and its absorbance read at 650 nm. The pH indicator dye is selected not to absorb in the 650 absorbance range. M-cresol purple is one such pH indicator dye which does not absorb at 650 nm.

The present invention further includes uses of kits for detecting an analyte in an enzyme linked assay and distinguishing a negative reaction from a false negative reaction in the assay comprising the above compositions. The above compositions can be provided in liquid or solid form. When provided in solid form, the composition can be in capsule, tablet, or powder form. In one embodiment, the kit comprises one or more first containers each of which contains a mixture of a chromogenic substrate and a pH indicator dye suitable for use with the chromogenic substrate and one or more second containers each of which contains a peroxide such as hydrogen peroxide or urea peroxide. In a second embodiment, the kit comprises one or more containers each of which contains a mixture of a chromogenic substrate, a peroxide such as hydrogen peroxide or urea peroxide, and a pH indicator suitable for use with the chromogenic substrate. In further embodiments of the above kits, the kits further contain a container containing a stop solution.

The above kits are particularly useful for use in peroxidase-linked immunoassays such as ELISAs. In the above kits, it is preferable that the chromogenic substrate is selected from the group consisting of OPD, OND, ABTS, 5AS, and TMB. It is further preferable that the pH indicator dye for acid stops be selected from the group consisting of cresol red, m-cresol purple, benzopurpurin 4B, metanil yellow, 4-phenylazodiphenylamine, malachite green, quinaldine red, orange IV, 2,2',2",4,4"-pentamethoxytriphenyl carbinol, and combinations thereof. It is further preferable that the pH indicator dye for use with 5AS be selected from the group consisting of phenolphthalein, thymolphthalein, alizarin yellow R, indigo carmine, and combinations thereof. In a most preferred embodiment, the chromogenic substrate is TMB or ABTS and the pH indicator dye is m-cresol purple. In further embodiments of the above kits, the acid stop is modified to inhibit further oxidization of the blue TMB to yellow.

The composition of the present invention can further be provided as a component of peroxidase-linked ELISAs. Therefore, the present invention further includes kits which comprise a peroxidase-linked ELISA for an analyte, one or more first containers each of which contains a mixture of a chromogenic substrate and a pH indicator dye suitable for use with the chromogenic substrate, and one or more second containers each of which contains a peroxide such as hydrogen peroxide or urea peroxide and kits which comprise a peroxidase-linked ELISA for an analyte and one or more containers each of which contains a mixture of a chromogenic substrate, a peroxide such as hydrogen peroxide or urea peroxide, and a pH indicator suitable for use with the chromogenic substrate. In further embodiments of the above kits, the kits further contain a container containing a stop solution.

In the above ELISA kits, it is preferable that the chromogenic substrate is selected from the group consisting of OPD, OND, ABTS, 5AS, and TMB. It is further preferable that the pH indicator dye for acid stops be selected from the group consisting of cresol red, m-cresol purple, benzopurpurin 4B, metanil yellow, 4-phenylazodiphenylamine, malachite green, quinaldine red, orange IV, 2,2',2",4,4"-pentamethoxytriphenyl carbinol, and combinations thereof. It is further preferable that the pH indicator dye for use with 5AS be selected from the group consisting of phenolphthalein, thymolphthalein, alizarin yellow R, indigo carmine, and combinations thereof. In a most preferred embodiment, the chromogenic substrate is TMB or ABTS and the pH indicator dye is m-cresol purple. In further embodiments of the above kits, the acid stop is modified to inhibit further oxidization of the blue TMB to yellow.

The above compositions and kits can further include one or more of the visible dyes disclosed in U.S. Patent No. 6,221,624 B1 to Lihme et al. Thus, the composition of the present invention can include compositions which comprise TMB, pH indicator dye, and a visible dye which is detectable by eye in the reaction vessel of the assay prior to addition of stop solution. The visible dye is selected so as to not substantially interfere with the absorbance of the oxidized TMB or with the oxidation of the TMB and not to substantially interfere with the change in color of the pH indicator dye. Preferably, the above composition further includes hydrogen peroxide. Therefore, in an assay, the visible dye renders the composition visible to the eye when it is added to an immunoassay; the chromogenic substrate enables reactions containing the analyte to be identified; and, the pH indicator dye enables negative reactions to be distinguished from false negative reactions.

The above method and kits can be adapted for use in alkaline phosphatase-based immunoassays such as alkaline phosphatase-based ELISAs. In the case of alkaline-phosphatase-based assays, the composition comprises p-nitrophenyl phosphate (PNNP) and a pH indicator dye. PNNP is hydrolyzed by alkaline phosphatase to p-nitrophenyloxide under basic conditions. Under typical reaction conditions the p-nitrophenyloxide is in equilibrium between its colorless benzenoid form and its yellow quinonoid form. Addition of a stop solution comprising a base such as sodium hydroxide (NaOH) or dipotassium phosphate (K₂HPO₄) to the reaction stops the reaction and shifts the equilibrium towards the quinonoid form. The quinonoid form has a maximum absorbances at 405 and 605 nm. Thus, suitable pH. indicator dye for the above reaction should be a dye which changes color at a high pH. Such pH indicator dyes include, but are not limited to, alizarin Yellow R, indigo carmine, and combinations thereof. In the case of alkaline phosphatdse-based immunoassays such as alkaline phosphatase-based ELISAs, the pH indicator dye comprising the composition can have a color which is detectable by eye when the composition is applied to the reaction vessel. The pH indicator dye does not need to be colorless or have a color which is essentially not detectable by eye when the composition is applied to the reaction vessel. However, the pH indicator dye comprising the composition must form a second color which is detectable by eye and has an absorbance at a second wavelength when a stop solution is added to the composition in the reaction vessel. The second wavelength cannot substantially interfere with detection of the first wavelength.

The above composition can be provided in one or more containers in a kit. The kit can further include a container containing a stop solution. The composition can further be included as a component of an alkaline phosphatase-linked ELISA kit for detecting particular analytes.

In particular embodiments of the above methods and kits, the composition can further include one or more of the dyes disclosed in U.S. Patent No. 6,221,624 B1 to Lihme et al.

### Example 1

This example shows that in an HRP reaction the absorbance of the m-cresol purple does not interfere with the absorbance of TMB.

The TMB was provided as a 100% water-based solution available as K-BLUE AQUEOUS, Neogen Corporation, Lansing, Michigan. To 10 mL of the TMB solution, 22 µg of m-cresol purple was added to make a positive/negative indicator solution. While the m-cresol purple was immediately soluble, to ensure even dispersal of the pH indicator dye, the mixture stirred for about five minutes. The amount of m-cresol purple added was an amount which was determined not to be visually detectable by eye. During stirring, the solution went from colorless to slight straw color. This change in color might have been because of the particular formulation of TMB used. Some TMB formulations will produce a color in solution. It was found to be preferably to add 11 µg of m-cresol purple to 10 mL of the TMB solution instead of 22 µg. At this concentration, the straw-yellow color was essentially indistinguishable to the eye but still produced a detectable rose-pink color upon addition of the stop solution. The performance of the solution containing m-cresol purple at a 2.2 µg/mL concentration was evaluated by incubating a 15 µL aliquot of the solution with a HRP in a quartz cuvette at room temperature. The spectra of the reaction was measured against water on a Shumadzu UV-1601 spectrophotometer.

Figure 1 shows the spectra of the reaction. The black line (-) is the spectra for a blank containing the solution but no HRP and the green line (-●-) is the spectra for the blank with acid added to it. As can be seen the blank was transparent between about 344 nm to greater than 686 nm. Visually, the solution was a light straw color, however, the color was attributable to the TMB formulation. However, adding acid to the solution oxidized the m-cresol purple which produced an absorbance at between about 524 to 542 nm. Visually, the solution was rose-pink. For the cuvette containing HRP, after 15 minutes, the TMB was oxidized to a blue color with a maximum absorbance at about 650 nm (blue line (-x-)). Note that there was no absorbance between about 524 to 542 nm. After adding acid to the reaction, the TMB was further oxidized to its diamine state which had a maximum absorbance at about 450 nm and the m-cresol was oxidized to produce an absorbance at between about 524 to 542 nm (red line (-|-)). Visually, the solution was a yellow and rose-pink blend.

### Example 2

In this example, performance of TMB with m-cresol purple in HRP reactions was compared to the performance of TMB alone. In this example, a direct competitive ELISA assay microwell format for detecting opiates was used (Opiate Group 100619 kit from Neogen Corp.). The assay operates on the basis of competition between opiates in the sample and opiate-HRP conjugates for a limited number of specific binding sites in precoated wells of microplates.

In this example, a 180 µL solution of opiate-HRP conjugate was added to each of the precoated wells of a microtiter plate to bind the opiate-HRP to the sites in the wells at room temperature. A set of sixteen wells was reserved to serve as controls. The controls were incubated with buffer blanks which did not contain opiate-HRP. After 45 minutes, the solution was removed from each of the wells and the wells washed three times with 300 µL of wash solution containing phosphate buffered saline and TWEEN. Next, 150 µL of a TMB solution (K-BLUE AQUEOUS, lot No. 020524)) was added to half of the microwells and 150 µL of TMB solution with m-cresol purple at 2.2 µg/mL was added to the other half of the wells. As the reaction progressed, the wells with HRP turned blue. After about 30 minutes, the absorbance at 650 nm for the wells was read using an ELISA reader.

The results from an eight-point standard curve for the wells which had been incubated with opiate-HRP showed that the color absorbance at 650 nm was similar for both sets of wells (1.244 OD for the wells with TMB and 1.201 OD for the wells with TMB with m-cresol purple) and the sensitivities were similar (I-50s of 0.16 ng/mL for the wells with TMB and 0.15 ng/mL for the wells with TMB with m-cresol purple). For the control wells, the OD₆₅₀ was 0.044 for the wells containing TMB and 0.047 for the wells containing TMB with m-cresol blue.

All of the above assays were then stopped by adding 50 µL of 1N HCL to each of the wells. All the wells which had been incubated with opiate-HRP developed a similar color at 450 nm. Acid added to eight of the control wells containing TMB resulted in colorless wells and acid added to the remaining eight control wells containing TMB and m-cresol purple resulted in light rose-pink wells. The absorbance of the rose-pink wells was measured at 562 nm because of the limited filter choices available with the ELISA reader. A 540 nm filter is preferred. The OD₄₅₀ was similar for both sets of controls (0.065 for the control wells containing TMB vs 0.079 for the control wells containing TMB with m-cresol purple. However, the OD₅₆₂ was higher for the control wells containing TMB with m-cresol purple (0.069 for the control wells containing TMB with m-cresol purple vs 0.041 for the control wells containing TMB). The OD₅₆₂ remained relatively stable even 4 hours after the acid had been added (0.079 for the control wells containing TMB with m-cresol purple vs 0.043 for the control wells containing TMB).

In addition to the above, the TMB and the TMB with m-cresol purple compositions were compared by adding two different volumes of each to 4 empty microwells on a standard Costar microtiter plate. The absorbance was measured on an ELISA plate reader using an air blank.

In the first experiment, 100 µL of each composition was added to the empty wells. The average OD₄₅₀ was 0.043 for the empty wells containing TMB and 0.063 for the empty wells containing TMB with m-cresol purple. The average OD₅₆₂ was 0.041 for the empty wells containing TMB and 0.037 for the empty wells containing TMB with m-cresol purple.

In the second experiment, 150 µL of each composition was added to the empty wells. The average OD₄₅₀ was 0.044 for the empty wells containing TMB and 0.078 for the empty wells containing TMB with m-cresol purple. The average OD₅₆₂ was 0.041 for the empty wells containing TMB and 0.040 for the empty wells containing TMB with m-cresol purple. The results show that the m-cresol purple is transparent at the indicator wavelength (OD₅₆₂) in the absence of acid.

### EXAMPLE 3

This example shows that using the m-cresol purple at a concentration of 1.1 µg/mL TMB substrate produced a rose-pink color upon addition of the stop solution even though the color of the solution was indistinguishable before hand.

The TMB solutions used were K-BLUE AQUEOUS, K-BLUE ENHANCED, and K-BLUE MAX (Neogen Corporation, Lansing, Michigan). To a well of a standard COSTAR microplate, 100 µL of TMB solution with or without 1.1 µg/mL m-cresol purple was added. Absorbances were read at 450 nm, 650 nm, and 540 nm. Afterwards, 100 µL of acid stop solution was added and the absorbances were read at 450 nm, 650 nm, and 540 nm. The optical densities were measured on a VMAX microplate reader (Molecular Devices Corporation, Sunnyvale, California) fitted with 450 nm, 650 nm, and 540 nm filters. The blank was air. The results are shown in Table 1.

**Table 1**

| Sample | 100 µL substrate | | | +100 µL acid (200 µL total) | | |
|---|---|---|---|---|---|---|
| K-BLUE AQUEOUS | OD₄₅₀ | OD₆₅₀ | OD₅₄₀ | OD₄₅₀ | OD₆₅₀ | OD₅₄₀ |
| Substrate | 0.044 | 0.037 | 0.039 | 0.040 | 0.035 | 0.036 |
| Substrate w/ indicator | 0.048 | 0.032 | 0.034 | 0.042 | 0.034 | 0.057 |

| K-BLUE ENHANCED | OD₄₅₀ | OD₆₅₀ | OD₅₄₀ | OD₄₅₀ | OD₆₅₀ | OD₅₄₀ |
|---|---|---|---|---|---|---|
| Substrate | 0.040 | 0.034 | 0.035 | 0.042 | 0.035 | 0.038 |
| Substrate w/ indicator | 0.052 | 0.036 | 0.037 | 0.042 | 0.032 | 0.057 |

| K-BLUE MAX | OD₄₅₀ | OD₆₅₀ | OD₅₄₀ | OD₄₅₀ | OD₆₅₀ | OD₅₄₀ |
|---|---|---|---|---|---|---|
| Substrate | 0.039 | 0.032 | 0.034 | 0.045 | 0.031 | 0.034 |
| Substrate w/ indicator | 0.049 | 0.032 | 0.035 | 0.042 | 0.030 | 0.055 |

For the volumes used, the OD₄₅₀ differences between substrate and substrate with indicator was not readily distinguishable to the eye even though the OD₅₄₀ differences were easily distinguishable to the eye as a light rose-pink color. Thus, this example shows that using the m-cresol purple at a concentration of about 1.1 µg/mL of substrate solution was able to produce a visible color and a detectable absorbance increase at 540 nm after the addition of acid even though a color was not distinguishable before the acid was added.

## Claims

1. A method for detecting an analyte in a peroxidase-based enzyme-linked immunosorbent assay (ELISA) and distinguishing a negative reaction from a false negative reaction in the ELISA, which comprises:
(a) providing a composition comprising: a mixture of a chromogenic substrate which is oxidizable by peroxide in a reaction for the ELISA catalyzed by the peroxidase of the ELISA to form a first color with an absorbance at a first wavelength, a pH indicator dye which forms a second color which is detectable by eye and has an absorbance at a second wavelength when a stop solution is added to the composition in a reaction vessel, and a peroxide;
(b) adding an aliquot of the composition to each of the reaction vessel for the ELISA to form a reaction mixture;
(c) incubating the reaction mixture for a time sufficient to oxidize the chromogenic substrate to the first color;
(d) adding the stop solution to the reaction mixture to stop the reaction and to generate the second color; and
(e) measuring spectrographically absorbances at the first and second wavelengths, wherein absorbance at the first wavelength indicates the detection of the analyte, absorbance at only the second wavelength indicates the negative reaction, and absence of absorbance at the first and second wavelengths indicates the false negative reaction or detecting the second color by eye wherein the second color indicates that the reaction mixture contains the composition and the stop solution.

2. The method of Claim 1 wherein the pH indicator is colorless or has a color which is essentially not detectable by eye when the composition is applied to a reaction vessel for the ELISA but which forms the second color which is detectable by eye and has the absorbance at the second wavelength when the stop solution is added to the composition in the reaction vessel.

3. The method of Claim 1 wherein the stop solution is an acid and the chromogenic substrate is selected from the group consisting of orthophenylenediamine (OPD), 2, 2'-azinobis- (3-ethyl-benzothiazoline-6-sulfonic acid) (ABTS), diaminobenzidine (DAB), 3, 3'dimethyloxybenzidine (ortho-dianisidine or ODN), and 3,3', 5, 5'-tetramethylbenzidine (TMB).

4. The method of Claim 1 wherein the stop solution is an acid and the chromogenic substrate is 2, 2'-azinobis- (3-ethyl-benzothiazoline-6-sulfonic acid) (ABTS).

5. The method of Claim 1 wherein the pH indicator dye is selected from the group consisting of cresol red, m-cresol purple, metanil yellow, 4-phenylazodiphenylamine, malachite green, orange IV, 2, 2', 2", 4, 4"-pentamethoxytriphenyl carbinol, and combinations thereof.

6. The method of Claim 1 wherein the stop solution is a base and the chromogenic substrate is 5-aminosalicylic acid (5AS).

7. The method of Claim 6 wherein the pH indicator dye is selected from the group consisting of phenolphthalein, thymolphthalein, alizarin Yellow R, indigo carmine, and combinations thereof.

8. The method of Claim 1 wherein the ELISA is selected from the group consisting of direct, indirect, and competitive ELISAs.

9. The method of Claim 1, wherein the chromogenic substrate is 3,3', 5,5'-tetramethylbenzidine (TMB).

10. The method of Claim 9 wherein the pH indicator dye is m-cresol purple.

11. The method of Claim 9 wherein the first wavelength is about 450 nm and the second wavelength is over 500 nm.

12. The method of Claim 3 or 4 wherein the acid is selected from the group consisting of HCL, H₂SO₄, and H₂PO₄.

13. Use of an enzyme immunoassay for distinguishing a negative reaction from a false negative reaction in the immunoassay and wherein the immunoassay is for colorimetric detection of an analyte of a type wherein a quantity of a first antibody is adsorbed to a solid support in a reaction vessel; a conjugate is formed between an immunologic reagent and a peroxidase; the conjugate is admixed with a sample to be tested for the analyte, the analyte binds to the first antibody and to the conjugate to form an immunologic complex in solid phase; and the quantity of the analyte is determined by measuring the reaction of the immunologic complex with a chromogenic substrate oxidizable by peroxide and the peroxidase comprising:
(a) providing a liquid solution comprising the chromogenic substrate, peroxide, and a pH indicator dye, wherein the chromogenic substrate is oxidizable to a first color with an absorbance at a first wavelength and the pH indicator dye produces a second color which is detectable by eye and which has an absorbance at a second wavelength when a stop solution is added to the reaction vessel containing the liquid solution;
(b) mixing the liquid solution with the immunologic complex in the reaction vessel to form a reaction mixture which oxidizes the chromogenic substrate to the first color;
(c) adding the stop solution to the reaction mixture in the reaction vessel to produce the second color, and,
(d) measuring spectrographically the absorbances at the first and second wavelengths, wherein absorbance at the first wavelength indicates detection of the analyte, absorbance at the second wavelength indicates a negative reaction, and an absence of absorbance at the first and second wavelength indicates a false negative reaction or detecting the second color by eye wherein the second color indicates that the reaction mixture contains the liquid solution and the stop solution.

14. Use of Claim 13 wherein the pH indicator is colorless or has a color which is essentially not detectable by eye when the composition is applied to a reaction vessel for the ELISA but which forms the second color which is detectable by eye and has the absorbance at the second wavelength when the stop solution is added to the composition in the reaction vessel.

15. Use of Claim 13 wherein the stop solution is an acid and the chromogenic substrate is selected from the group consisting of ortho-phenylenediamine (OPD), 2, 2'-azinobis- (3-ethyl-benzothiazoline-6-sulfonic acid) (ABTS), diaminobenzidine (DAB), 3, 3'dimethyloxybenzidine (ortho-dianisidine or ODN), and 3, 3', 5, 5'-tetramethylbenzidine (TMB).

16. Use of Claim 13 wherein the stop solution is an acid and the chromogenic substrate is 2,2'-azinobis- (3-ethyl-benzothiazoline-6-sulfonic acid) (ABTS).

17. Use of Claim 13 wherein the pH indicator dye is selected from the group consisting of cresol red, m-cresol purple, metanil yellow, 4- phenylazodiphenylamine, malachite green, orange IV, 2, 2', 2", 4, 4"-pentamethoxytriphenyl carbinol, and combinations thereof.

18. Use of Claim 13 wherein the stop solution is a base and the chromogenic substrate is 5-aminosalicylic acid (5AS).

19. Use of Claim 18 wherein the pH indicator dye is selected from the group consisting of phenolphthalein, thymolphthalein, alizarin Yellow R, indigo carmine, and combinations thereof.

20. Use of claim 13, wherein the chromogenic substrate is 3,3', 5,5'-tetramethylbenzidine (TMB).

21. Use of Claim 20 wherein the pH indicator dye is m-cresol purple.

22. Use of Claim 20 wherein the first wavelength is about 450 nm and the second wavelength is over 500 nm.

23. Use of Claim 15 or 16 wherein the acid is selected from the group consisting of HCL, H₂SO₄, and H₂PO₄.

24. Use of an enzyme immunoassay for distinguishing a negative reaction from a false negative reaction in the immunoassay and wherein the immunoassay is for colorimetric detection of an antibody of a type wherein a quantity of an analyte is adsorbed to a solid support in a reaction vessel; a conjugate is formed between an immunologic reagent and a peroxidase; the conjugate is admixed with a sample to be tested for the antibody, the antibody binds to the analyte and to the conjugate to form an immunologic complex in solid phase; and the quantity of the antibody is determined by measuring the reaction of the immunologic complex with a chromogenic substrate oxidizable by peroxide and the peroxidase comprising:
(a) providing a liquid solution comprising the chromogenic substrate, peroxide, and a pH indicator dye, wherein the chromogenic substrate is oxidizable to a first color with an absorbance at a first wavelength and the pH indicator dye produces a second color which is detectable by eye and which has an absorbance at a second wavelength when a stop solution is added to the reaction vessel containing the liquid solution;
(b) mixing the liquid solution with the immunologic complex in the reaction vessel to form a reaction mixture which oxidizes the chromogenic substrate to the first color;
(c) adding the stop solution to the reaction mixture in the reaction vessel to produce the second color; and,
(d) measuring spectrographically the absorbances at the first and second wavelengths, wherein absorbance at the first wavelength indicates detection of the analyte, absorbance at the second wavelength indicates a negative reaction, and an absence of absorbance at the first and second wavelength indicates a false negative reaction or detecting the second color by eye wherein the second color indicates that the reaction mixture contains the liquid solution and the stop solution.

25. Use of Claim 24 wherein the pH indicator is colorless or has a color which is essentially not detectable by eye when the composition is applied to a reaction vessel for the ELISA but which forms the second color which is detectable by eye and has the absorbance at the second wavelength when the stop solution is added to the composition in the reaction vessel.

26. Use of Claim 24 wherein the stop solution is an acid and the chromogenic substrate is selected from the group consisting of ortho-phenylenediamine (OPD), 2, 2'-azinobis-(3-ethyl-benzothiazoline-6 sulfonic acid) (ABTS), diaminobenzidine (DAB), 3,3'dimethyloxybenzidine (ortho-dianisidine or ODN), and 3, 3', 5,5'-tetramethylbenzidine (TMB).

27. Use of Claim 24 wherein the stop solution is an acid and the chromogenic substrate is 2, 2'-azinobis-(3-ethyl-benzothiazoline-6-sulfonic acid) (ABTS).

28. Use of Claim 24 wherein the pH indicator dye is selected from the group consisting of cresol red, m-cresol purple, metanil yellow, 4-phenylazodiphenylamine, malachite green, orange IV, 2, 2', 2", 4, 4"-pentamethoxytriphenyl carbinol, and combinations thereof.

29. Use of Claim 24 wherein the stop solution is a base and the chromogenic substrate is 5-aminosalicylic acid (5AS).

30. Use of Claim 24 wherein the pH indicator dye is selected from the group consisting of phenolphthalein, thymolphthalein, alizarin Yellow R, indigo carmine, and combinations thereof.

31. Use of an enzyme immunoassay of claim 24, wherein the chromogenic substrate is 3,3',5,5'-tetramethylbenzidine (TMB).

32. Use of Claim 31 wherein the pH indicator dye is m-cresol purple.

33. Use of Claim 31 wherein the first wavelength is about 450 nm and the second wavelength is over 500 nm.

34. Use of Claim 31 wherein the acid is selected from the group consisting of HCL, H₂SO₄, and H₂PO₄.

35. Use of a kit for detecting an analyte in an enzyme linked assay and distinguishing a negative reaction from a false negative reaction in the assay, which comprises:
one or more first containers each of which contains a mixture of a chromogenic substrate with an absorbance at a first wavelength, a pH indicator dye suitable for use with the chromogenic substrate, which produces a color detectable by eye which has an absorbance at a second wavelength when an acid or base stop solution is added to the mixture in the reaction vessel, and a peroxide,
wherein the absorbances at the first and second wavelengths are measured spectrographically,
wherein absorbance at the first wavelength indicates detection of the analyte, absorbance at the second wavelength indicates a negative reaction, and an absence of absorbance at the first and second wavelength indicates a false negative reaction or detecting the second color by eye wherein the second color indicates that the reaction mixture contains the liquid solution and the stop solution.

36. Use of Claim 35 wherein the pH indicator is colorless or has a color which is essentially not detectable by eye when the composition is applied to a reaction vessel for the assay but which forms the second color which is detectable by eye and has the absorbance at the second wavelength when the stop solution is added to the composition in the reaction vessel.

37. Use of Claim 35 wherein the first container contains the chromogenic substrate and the pH indicator dye and the peroxide is contained in a second container.

38. Use of Claim 35 or 36 wherein the kit further includes a third container containing an acid or base stop solution.

39. Use of Claim 35 wherein the chromogenic substrate is selected from the group consisting of ortho-phenylenediamine (OPD), 2, 2'-azinobis-(3-ethylbenzothiazoline-6-sulfonic acid) (ABTS), diaminobenzidine (DAB), 3,3'-dimethyloxybenzidine (orthodianisidine or ODN), and 3, 3', 5, 5'-tetramethylbenzidine (TMB).

40. Use of Claim 35 wherein the pH indicator dye is selected from the group consisting of cresol red, m-cresol purple, metanil yellow, 4-phenylazodiphenylamine, malachite green, quinaldine red, orange IV, 2, 2', 2", 4, 4"-pentamethoxytriphenyl carbinol, and combinations thereof.

41. Use of Claim 35 wherein the chromogenic substrate is 5-aminosalicylic acid (5AS).

42. Use of Claim 41 wherein the pH indicator dye is selected from the group consisting of phenolphthalein, thymolphthalein, alizarin yellow R, indigo carmine, and combinations thereof.

43. Use of Claim 35 wherein the pH indicator dye is m-cresol purple and the chromogenic substrate is selected from the group consisting of 3,3', 5, 5'- tetramethylbenzidine (TMB) and 2, 2'-azinobis-(3-ethyl-benzothiazoline-6-sulfonic acid) (ABTS).

44. Use of Claim 35 wherein the enzyme-linked assay is an enzyme-linked immunosorbent assay (ELISA).

45. Use of claim 35, wherein the chromogenic substrate is selected from the group consisting of ortho-phenylenediamine (OPD), 2, 2'- azinobis-(3-ethyl-benzothiazoline-6-sulfonic acid) (ABTS), diaminobenzidine (DAB), and 3,3'-dimethyloxybenzidine (ortho-dianisidine or ODN) which is converted by an enzyme in a reaction vessel for the assay to a first color.

46. Use of Claim 45 wherein the chromogenic substrate is p-nitrophenyl phosphate.

47. Use of Claim 46 wherein the pH indicator dye is selected from the group consisting of alizarin Yellow R, indigo carmine, and combinations thereof.

48. Use of a composition for use in an enzyme-linked immunosorbent assay (ELISA) and for distinguishing a negative reaction from a false negative reaction in the ELISA, which comprises in a mixture:
(a) a chromogenic substrate which is oxidizable by a peroxide generated in the ELISA in a reaction mixture to which a stop solution is then added to stop the reaction to form a first color with an absorbance at a first wavelength; and
(b) a pH indicator dye which is colorless or has a color which is essentially not detectable by eye when the composition is applied to a reaction vessel for the ELISA but which produces a second color detectable by eye and which has an absorbance at a second wavelength when an acid or base stop solution is added to the composition in the reaction vessel, wherein the absorbances at the first and second wavelengths are measured spectrographically, wherein absorbance at the first wavelength indicates detection of the analyte, absorbance at the second wavelength indicates a negative reaction, and an absence of absorbance at the first and second wavelength indicates a false negative reaction or detecting the second color by eye wherein the second color indicates that the reaction mixture contains the liquid solution and the stop solution.

49. Use of Claim 48 wherein the chromogenic substrate is selected from the group consisting of ortho-phenylenediamine (OPD), 2,2'-azinobis-(3-ethyl-benzothiazoline-6-sulfonic acid) (ABTS), diaminobenzidine (DAB), and 3,3'-dimethyloxybenzidine (ortho-dianisidine or ODN).

50. Use of Claim 49 wherein the pH indicator dye is selected from the group consisting of cresol red, m-cresol purple, metanil yellow, 4-phenylazodiphenylamine, malachite green, quinaldine red, orange IV, 2,2', 2", 4, 4"-pentamethoxytriphenyl carbinol, and combinations thereof.

51. Use of Claim 48 wherein the chromogenic substrate is 5-aminosalicylic acid (5AS).

52. Use of Claim 51 wherein the pH indicator dye is selected from the group consisting of phenolphthalein, thymolphthalein, alizarin yellow R, indigo carmine, and combinations thereof.

53. Use of Claim 48 wherein the pH indicator dye is m-cresol purple and the chromogenic substrate is 2, 2'-azinobis- (3-ethyl-benzothiazoline-6-sulfonic acid) (ABTS).

54. Use of claim 48, wherein the chromogenic substrate is 3, 3', 5, 5'-tetramethylbenzidine (TMB).

55. Use of Claim 54 wherein the pH indicator dye is m-cresol purple.

56. A method for detecting an analyte in a enzyme-linked assay and distinguishing a negative reaction from a false negative reaction in the assay, which comprises:
(a) providing a composition comprising a mixture of a chromogenic substrate selected from the group consisting of ortho-phenylenediamine (OPD), 2, 2'-azinobis-(3-ethyl-benzothiazoline-6-sulfonic acid) (ABTS), diaminobenzidine (DAB), and 3,3'-dimethyloxybenzidine (ortho-dianisidine or ODN) which is converted by an enzyme in a reaction vessel for the assay to a first color which has an absorbance at a first wavelength and a pH indicator dye which produces a second color detectable by eye and which has an absorbance at a second wavelength when an acid or base stop solution is added to the composition in the reaction vessel;
(b) adding an aliquot of the composition to the reaction vessel for the enzyme-linked assay to form a reaction mixture;
(c) incubating the reaction mixture for a time sufficient to produce the first color;
(d) adding the stop solution to the reaction mixture to stop the reaction and to generate the second color with the second wavelength; and
(e) measuring spectrographically absorbances at the first and second wavelengths, wherein absorbance at the first wavelength indicates the detection of the analyte, absorbance at only the second wavelength indicates the negative reaction, and absence of absorbance at the first and second wavelengths indicates the false negative reaction or detecting the second color by eye wherein the second color indicates that the reaction mixture contains the composition and the stop solution.

57. The method of Claim 56 wherein the enzyme is an alkaline phosphatase, the chromogenic substrate is p-nitrophenyl phosphate, and the stop solution is a base.

58. The method of Claim 57 wherein the pH indicator dye is selected from the group consisting of alizarin Yellow R, indigo carmine, and combinations thereof.

59. The method of Claim 56 wherein the assay is an enzyme-linked immunosorbent assay (ELISA).

60. The method of Claim 56 or 59 wherein the assay is selected from the group consisting of direct, indirect, and competitive assay.

61. The method of Claim 60 wherein the pH indicator dye is selected from the group consisting of cresol red, m-cresol purple, metanil yellow, 4-phenylazodiphenylamine, malachite green, orange IV, 2,2', 2", 4, 4"-pentamethoxytriphenyl carbinol, and combinations thereof.

62. Use of a composition for use in an enzyme-linked assay and for distinguishing a negative reaction from a false negative reaction in the assay, which comprises in a mixture:
(a) a chromogenic substrate is selected from the group consisting of orthophenylenediamine (OPD), 2,2'-azinobis-(3-ethyl-benzothiazoline-6-sulfonic acid) (ABTS), diaminobenzidine (DAB), and 3, 3'-dimethyloxybenzidine (ortho-dianisidine or ODN) which is converted by an enzyme in a reaction vessel for the assay to a first color with an absorbance at a first wavelength; and
(b) a pH indicator dye which produces a second color detectable by eye and has an absorbance at a second wavelength when an acid or base stop solution is added to the composition in the reaction vessel;
wherein the absorbances at the first and second wavelengths are measured spectrographically, wherein absorbance at the first wavelength indicates detection of the analyte, absorbance at the second wavelength indicates a negative reaction, and an absence of absorbance at the first and second wavelength indicates a false negative reaction or detecting the second color by eye wherein the second color indicates that the reaction mixture contains the liquid solution and the stop solution.

63. Use of Claim 62 wherein the chromogenic substrate is p-nitrophenyl phosphate.

64. Use of Claim 63 wherein the pH indicator dye is selected from the group consisting of alizarin Yellow R, indigo carmine, and combinations thereof.

65. Use of Claim 62 wherein the pH indicator dye is selected from the group consisting of cresol red, m-cresol purple, metanil yellow, 4-phenylazodiphenylamine, malachite green, orange IV, 2, 2', 2", 4, 4"-pentamethoxytriphenyl carbinol, and combinations thereof.

66. Use of an enzyme immunoassay for distinguishing a negative reaction from a false negative reaction in the immunoassay and wherein the immunoassay is for colorimetric detection of an analyte of a type wherein a quantity of a first antibody is adsorbed to a solid support in a reaction vessel; a conjugate is formed between an immunologic reagent and a peroxidase; the conjugate is admixed with a sample to be tested for the analyte, the analyte binds to the first antibody and to the conjugate to form an immunologic complex in solid phase; and the quantity of the analyte is determined by measuring the reaction of the immunologic complex with a chromogenic substrate oxidizable by peroxide and the peroxidase comprising:
(a) providing a liquid solution comprising the chromogenic substrate, peroxide, and a pH indicator dye, wherein the chromogenic substrate is TMB or OPD and is oxidizable to a first color with an absorbance at a first wavelength when a reaction mixture is formed between the liquid solution and the immunologic complex and is further oxidizable from the first color to a second color with an absorbance at a second wavelength when a stop solution is added to the reaction vessel and the pH indicator dye produces a third color which is detectable by eye and which has an absorbance at a third wavelength when a stop solution is added to the reaction vessel containing the liquid solution;
(b) mixing the liquid solution with the immunologic complex in the reaction vessel to form a reaction mixture which oxidizes the chromogenic substrate to the first color;
(c) adding the stop solution to the reaction mixture in the reaction vessel to produce the second and third colors; and,
(d) measuring spectrographically the absorbances at the first, second and third wavelengths, wherein a positive reaction is detected by absorbance at the second wavelength, reactions which have no absorbance at the first or second wavelength, but have absorbance at the third wavelength are scored as negative, and wherein all reactions with no absorbance at the third wavelength are scored as false negatives.

67. Use of an enzyme immunoassay for distinguishing a negative reaction from a false negative reaction in the immunoassay and wherein the immunoassay is for colorimetric detection of an antibody of a type wherein a quantity of an analyte is adsorbed to a solid support in a reaction vessel; a conjugate is formed between an immunologic reagent and a peroxidase; the conjugate is admixed with a sample to be tested for the antibody, the antibody binds to the analyte and to the conjugate to form an immunologic complex in solid phase; and the quantity of the antibody is determined by measuring the reaction of the immunologic complex with a chromogenic substrate oxidizable by peroxide and the peroxidase comprising:
(a) providing a liquid solution comprising the chromogenic substrate, peroxide, and a pH indicator dye, wherein the chromogenic substrate is TMB or OPD and is oxidizable to a first color with an absorbance at a first wavelength when a reaction mixture is formed between the liquid solution and the immunologic complex and is further oxidizable from the first color to a second color with an absorbance at a second wavelength when a stop solution is added to the reaction vessel and the pH indicator dye produces a third color which is detectable by eye and which has an absorbance at a third wavelength when a stop solution is added to the reaction vessel containing the liquid solution;
(b) mixing the liquid solution with the immunologic complex in the reaction vessel to form a reaction mixture which oxidizes the chromogenic substrate to the first color;
(c) adding the stop solution to the reaction mixture in the reaction vessel to produce the second and third colors; and,
(d) measuring spectrographically the absorbances at the first, second and third wavelengths, wherein a positive reaction is detected by absorbance at the second wavelength, reactions which have no absorbance at the first or second wavelength, but have absorbance at the third wavelength are scored as negative, and wherein all reactions with no absorbance at the third wavelength are scored as false negatives.

## Patentansprüche

1. Verfahren zum Nachweisen eines Analyten in einem Peroxidase-gestützten enzymgekoppelten Immunoadsorptionsassay (ELISA) und Unterscheiden einer negativen Reaktion von einer falsch negativen Reaktion in dem ELISA, welches umfasst:
(a) Bereitstellen einer Zusammensetzung, umfassend ein Gemisch aus einem chromogenen Substrat, das in einer durch die Peroxidase des ELISAs katalysierten Reaktion für den ELISA durch Peroxid oxidierbar ist, um eine erste Farbe mit einer Absorption bei einer ersten Wellenlänge zu bilden, einem pH-Indikatorfarbstoff, der eine zweite Farbe, die mit dem Auge nachweisbar ist und eine Absorption bei einer zweiten Wellenlänge aufweist, bildet, wenn eine Stopplösung zu der Zusammensetzung in einem Reaktionsgefäß gegeben wird, und einem Peroxid;
(b) Zugeben eines Aliquots der Zusammensetzung zu jedem Reaktionsgefäß für den ELISA, um ein Reaktionsgemisch zu bilden;
(c) Inkubieren des Reaktionsgemisches über eine Zeit hinweg, die ausreicht, um das chromogene Substrat zu der ersten Farbe zu oxidieren;
(d) Zugeben der Stopplösung zu dem Reaktionsgemisch, um die Reaktion zu beenden und die zweite Farbe zu erzeugen; und
(e) spektrographisches Messen von Absorptionen bei der ersten und zweiten Wellenlänge, wobei Absorption bei der ersten Wellenlänge den Nachweis des Analyten anzeigt, Absorption nur bei der zweiten Wellenlänge die negative Reaktion anzeigt und Abwesenheit von Absorption bei der ersten und zweiten Wellenlänge die falsch negative Reaktion anzeigt, oder Nachweisen der zweiten Farbe mit dem Auge, wobei die zweite Farbe anzeigt, dass das Reaktionsgemisch die Zusammensetzung und die Stopplösung enthält.

2. Verfahren nach Anspruch 1, wobei der pH-Indikator farblos ist oder eine Farbe aufweist, die im Wesentlichen nicht mit dem Auge nachweisbar ist, wenn die Zusammensetzung auf ein Reaktionsgefäß für den ELISA angewendet wird, der aber die zweite Farbe, die mit dem Auge nachweisbar ist und die Absorption bei der zweiten Wellenlänge aufweist, bildet, wenn die Stopplösung zu der Zusammensetzung in dem Reaktionsgefäß gegeben wird.

3. Verfahren nach Anspruch 1, wobei es sich bei der Stopplösung um eine Säure handelt und das chromogene Substrat aus der Gruppe ausgewählt ist, bestehend aus ortho-Phenylendiamin (OPD), 2, 2'-Azinobis-(3-ethylbenzothiazolin-6-sulfonsäure) (ABTS), Diaminobenzidin (DAB), 3, 3'-Dimethyloxybenzidin (ortho-Dianisidin oder ODN) und 3, 3', 5, 5'-Tetramethylbenzidin (TMB).

4. Verfahren nach Anspruch 1, wobei es sich bei der Stopplösung um eine Säure und bei dem chromogenen Substrat um 2, 2'-Azinobis-(3-ethylbenzothiazolin-6-sulfonsäure) (ABTS) handelt.

5. Verfahren nach Anspruch 1, wobei der pH-Indikatorfarbstoff aus der Gruppe ausgewählt ist, bestehend aus Cresolrot, m-Cresolpurpur, Metanilgelb, 4-Phenylazodiphenylamin, Malachitgrün, Orange IV, 2, 2', 2", 4, 4"-Pentamethoxytriphenylcarbinol und Kombinationen davon.

6. Verfahren nach Anspruch 1, wobei es sich bei der Stopplösung um eine Base und bei dem chromogenen Substrat um 5-Aminosalicylsäure (5AS) handelt.

7. Verfahren nach Anspruch 6, wobei der pH-Indikatorfarbstoff aus der Gruppe ausgewählt ist, bestehend aus Phenolphthalein, Thymolphthalein, Alizaringelb R, Indigocarmin und Kombinationen davon.

8. Verfahren nach Anspruch 1, wobei der ELISA aus der Gruppe ausgewählt ist, bestehend aus direkten, indirekten und kompetitiven ELISAs.

9. Verfahren nach Anspruch 1, wobei es sich bei dem chromogenen Substrat um 3, 3', 5, 5'-Tetramethylbenzidin (TMB) handelt.

10. Verfahren nach Anspruch 9, wobei es sich bei dem pH-Indikatorfarbstoff um m-Cresolpurpur handelt.

11. Verfahren nach Anspruch 9, wobei es sich bei der ersten Wellenlänge um etwa 450 nm und bei der zweiten Wellenlänge um über 500 nm handelt.

12. Verfahren nach Anspruch 3 oder 4, wobei die Säure aus der Gruppe ausgewählt ist, bestehend aus HCl, H₂SO₄ und H₂PO₄.

13. Verwendung eines Enzymimmunoassays zum Unterscheiden einer negativen Reaktion von einer falsch negativen Reaktion in dem Immunoassay und wobei der Immunoassay zum colorimetrischen Nachweis eines Analyten bestimmt ist, derart, dass eine Menge eines ersten Antikörpers an eine feste Unterlage in einem Reaktionsgefäß adsorbiert wird; ein Konjugat zwischen einem immunologischen Reagenz und einer Peroxidase gebildet wird; das Konjugat mit einer auf den Analyten zu testenden Probe gemischt wird, der Analyt an den ersten Antikörper und an das Konjugat bindet, um einen immunologischen Komplex in fester Phase zu bilden; und die Menge des Analyten durch Messen der Reaktion des immunologischen Komplexes mit einem durch Peroxid oxidierbaren chromogenen Substrat und der Peroxidase bestimmt wird, umfassend:
(a) Bereitstellen einer flüssigen Lösung, umfassend das chromogene Substrat, Peroxid und einen pH-Indikatorfarbstoff, wobei das chromogene Substrat zu einer ersten Farbe mit einer Absorption bei einer ersten Wellenlänge oxidierbar ist und der pH-Indikatorfarbstoff eine zweite Farbe produziert, die mit dem Auge nachweisbar ist und die eine Absorption bei einer zweiten Wellenlänge aufweist, wenn eine Stopplösung zu dem Reaktionsgefäß gegeben wird, welches die flüssige Lösung enthält;
(b) Mischen der flüssigen Lösung mit dem immunologischen Komplex in dem Reaktionsgefäß, um ein Reaktionsgemisch zu bilden, welches das chromogene Substrat zu der ersten Farbe oxidiert;
(c) Zugeben der Stopplösung zu dem Reaktionsgemisch in dem Reaktionsgefäß, um die zweite Farbe zu produzieren; und
(d) spektrographisches Messen der Absorptionen bei der ersten und zweiten Wellenlänge, wobei Absorption bei der ersten Wellenlänge den Nachweis des Analyten anzeigt, Absorption bei der zweiten Wellenlänge eine negative Reaktion anzeigt und eine Abwesenheit von Absorption bei der ersten und zweiten Wellenlänge eine falsch negative Reaktion anzeigt, oder Nachweisen der zweiten Farbe mit dem Auge, wobei die zweite Farbe anzeigt, dass das Reaktionsgemisch die flüssige Lösung und die Stopplösung enthält.

14. Verwendung nach Anspruch 13, wobei der pH-Indikator farblos ist oder eine Farbe aufweist, die im Wesentlichen nicht mit dem Auge nachweisbar ist, wenn die Zusammensetzung auf ein Reaktionsgefäß für den ELISA angewendet wird, der aber die zweite Farbe, die mit dem Auge nachweisbar ist und die Absorption bei der zweiten Wellenlänge aufweist, bildet, wenn die Stopplösung zu der Zusammensetzung in dem Reaktionsgefäß gegeben wird.

15. Verwendung nach Anspruch 13, wobei es sich bei der Stopplösung um eine Säure handelt und das chromogene Substrat aus der Gruppe ausgewählt ist, bestehend aus ortho-Phenylendiamin (OPD), 2, 2'-Azinobis-(3-ethylbenzothiazolin-6-sulfonsäure) (ABTS), Diaminobenzidin (DAB), 3, 3'-Dimethyloxybenzidin (ortho-Dianisidin oder ODN) und 3, 3', 5, 5'-Tetramethylbenzidin (TMB).

16. Verwendung nach Anspruch 13, wobei es sich bei der Stopplösung um eine Säure und bei dem chromogenen Substrat um 2, 2'-Azinobis-(3-ethylbenzothiazolin-6-sulfonsäure) (ABTS) handelt.

17. Verwendung nach Anspruch 13, wobei der pH-Indikatorfarbstoff aus der Gruppe ausgewählt ist, bestehend aus Cresolrot, m-Cresolpurpur, Metanilgelb, 4-Phenylazodiphenylamin, Malachitgrün, Orange IV, 2, 2', 2", 4, 4"-Pentamethoxytriphenylcarbinol und Kombinationen davon.

18. Verwendung nach Anspruch 13, wobei es sich bei der Stopplösung um eine Base und bei dem chromogenen Substrat um 5-Aminosalicylsäure (5AS) handelt.

19. Verwendung nach Anspruch 18, wobei der pH-Indikatorfarbstoff aus der Gruppe ausgewählt ist, bestehend aus Phenolphthalein, Thymolphthalein, Alizaringelb R, Indigocarmin und Kombinationen davon.

20. Verwendung nach Anspruch 13, wobei es sich bei dem chromogenen Substrat um 3, 3', 5, 5'-Tetramethylbenzidin (TMB) handelt.

21. Verwendung nach Anspruch 20, wobei es sich bei dem pH-Indikatorfarbstoff um m-Cresolpurpur handelt.

22. Verwendung nach Anspruch 20, wobei es sich bei der ersten Wellenlänge um etwa 450 nm und bei der zweiten Wellenlänge um über 500 nm handelt.

23. Verwendung nach Anspruch 15 oder 16, wobei die Säure aus der Gruppe ausgewählt ist, bestehend aus HCl, H₂SO₄ und H₂PO₄.

24. Verwendung eines Enzymimmunoassays zum Unterscheiden einer negativen Reaktion von einer falsch negativen Reaktion in dem Immunoassay und wobei der Immunoassay zum colorimetrischen Nachweis eines Antikörpers bestimmt ist, derart, dass eine Menge eines Analyten an eine feste Unterlage in einem Reaktionsgefäß adsorbiert wird; ein Konjugat zwischen einem immunologischen Reagenz und einer Peroxidase gebildet wird; das Konjugat mit einer auf den Antikörper zu testenden Probe gemischt wird, der Antikörper an den Analyten und an das Konjugat bindet, um einen immunologischen Komplex in fester Phase zu bilden; und die Menge des Antikörpers durch Messen der Reaktion des immunologischen Komplexes mit einem durch Peroxid oxidierbaren chromogenen Substrat und der Peroxidase bestimmt wird, umfassend:
(a) Bereitstellen einer flüssigen Lösung, umfassend das chromogene Substrat, Peroxid und einen pH-Indikatorfarbstoff, wobei das chromogene Substrat zu einer ersten Farbe mit einer Absorption bei einer ersten Wellenlänge oxidierbar ist und der pH-Indikatorfarbstoff eine zweite Farbe produziert, die mit dem Auge nachweisbar ist und die eine Absorption bei einer zweiten Wellenlänge aufweist, wenn eine Stopplösung zu dem Reaktionsgefäß gegeben wird, welches die flüssige Lösung enthält;
(b) Mischen der flüssigen Lösung mit dem immunologischen Komplex in dem Reaktionsgefäß, um ein Reaktionsgemisch zu bilden, welches das chromogene Substrat zu der ersten Farbe oxidiert;
(c) Zugeben der Stopplösung zu dem Reaktionsgemisch in dem Reaktionsgefäß, um die zweite Farbe zu produzieren; und
(d) spektrographisches Messen der Absorptionen bei der ersten und zweiten Wellenlänge, wobei Absorption bei der ersten Wellenlänge den Nachweis des Analyten anzeigt, Absorption bei der zweiten Wellenlänge eine negative Reaktion anzeigt und eine Abwesenheit von Absorption bei der ersten und zweiten Wellenlänge eine falsch negative Reaktion anzeigt, oder Nachweisen der zweiten Farbe mit dem Auge, wobei die zweite Farbe anzeigt, dass das Reaktionsgemisch die flüssige Lösung und die Stopplösung enthält.

25. Verwendung nach Anspruch 24, wobei der pH-Indikator farblos ist oder eine Farbe aufweist, die im Wesentlichen nicht mit dem Auge nachweisbar ist, wenn die Zusammensetzung auf ein Reaktionsgefäß für den ELISA angewendet wird,
der aber die zweite Farbe, die mit dem Auge nachweisbar ist, bildet und die Absorption bei der zweiten Wellenlänge aufweist, wenn die Stopplösung zu der Zusammensetzung in dem Reaktionsgefäß gegeben wird.

26. Verwendung nach Anspruch 24, wobei es sich bei der Stopplösung um eine Säure handelt und das chromogene Substrat aus der Gruppe ausgewählt ist, bestehend aus ortho-Phenylendiamin (OPD), 2, 2'-Azinobis-(3-ethylbenzothiazolin-6-sulfonsäure) (ABTS), Diaminobenzidin (DAB), 3, 3'-Dimethyloxybenzidin (ortho-Dianisidin oder ODN) und 3, 3', 5, 5'-Tetramethylbenzidin (TMB).

27. Verwendung nach Anspruch 24, wobei es sich bei der Stopplösung um eine Säure und bei dem chromogenen Substrat um 2, 2'-Azinobis-(3-ethylbenzothiazolin-6-sulfonsäure) (ABTS) handelt.

28. Verwendung nach Anspruch 24, wobei der pH-Indikatorfarbstoff aus der Gruppe ausgewählt ist, bestehend aus Cresolrot, m-Cresolpurpur, Metanitgelb, 4-Phenylazodiphenylamin, Malachitgrün, Orange IV, 2, 2', 2",4 ,4"-Pentamethoxytriphenylcarbinol und Kombinationen davon.

29. Verwendung nach Anspruch 24, wobei es sich bei der Stopplösung um eine Base und bei dem chromogenen Substrat um 5-Aminosalicylsäure (5AS) handelt.

30. Verwendung nach Anspruch 24, wobei der pH-Indikatorfarbstoff aus der Gruppe ausgewählt ist, bestehend aus Phenolphthalein, Thymolphthalein, Alizaringelb R, Indigocarmin und Kombinationen davon.

31. Verwendung eines Enzymimmunoassays nach Anspruch 24, wobei es sich bei dem chromogenen Substrat um 3, 3', 5, 5'-Tetramethylbenzidin (TMB) handelt.

32. Verwendung nach Anspruch 31, wobei es sich bei dem pH-Indikatorfarbstoff um m-Cresolpurpur handelt.

33. Verwendung nach Anspruch 31, wobei es sich bei der ersten Wellenlänge um etwa 450 nm und bei der zweiten Wellenlänge um über 500 nm handelt.

34. Verwendung nach Anspruch 31, wobei die Säure aus der Gruppe ausgewählt ist, bestehend aus HCl, H₂SO₄ und H₂PO₄.

35. Verwendung eines Kits zum Nachweisen eines Analyten in einem enzymgekoppelten Assay und Unterscheiden einer negativen Reaktion von einer falsch negativen Reaktion in dem Assay, welcher umfasst:
einen oder mehrere erste Behälter, von denen jeder ein Gemisch aus einem chromogenen Substrat mit einer Absorption bei einer ersten Wellenlänge, einem zur Verwendung mit dem chromogenen Substrat geeigneten pH-Indikatorfarbstoff, der eine mit dem Auge nachweisbare Farbe, die eine Absorption bei einer zweiten Wellenlänge aufweist, produziert, wenn eine Säure- oder Basen-Stopplösung zu dem Gemisch in dem Reaktionsgefäß gegeben wird,
und einem Peroxid enthält,
wobei die Absorptionen bei der ersten und zweiten Wellenlänge spektrographisch gemessen werden,
wobei Absorption bei der ersten Wellenlänge den Nachweis des Analyten anzeigt, Absorption bei der zweiten Wellenlänge eine negative Reaktion anzeigt und eine Abwesenheit von Absorption bei der ersten und zweiten Wellenlänge eine falsch negative Reaktion anzeigt, oder Nachweisen der zweiten Farbe mit dem Auge, wobei die zweite Farbe anzeigt, dass das Reaktionsgemisch die flüssige Lösung und die Stopplösung enthält.

36. Verwendung nach Anspruch 35, wobei der pH-Indikator farblos ist oder eine Farbe aufweist, die im Wesentlichen licht mit dem Auge nachweisbar ist, wenn die Zusammensetzung auf ein Reaktionsgefäß für den Assay angewendet wird, der aber die zweite Farbe, die mit dem Auge nachweisbar ist und die Absorption bei der zweiten Wellenlänge aufweist, bildet, wenn die Stopplösung zu der Zusammensetzung in dem Reaktionsgefäß gegeben wird.

37. Verwendung nach Anspruch 35, wobei der erste Behälter das chromogene Substrat und den pH-Indikatorfarbstoff enthält und das Peroxid in einem zweiten Behälter enthalten ist.

38. Verwendung nach Anspruch 35 oder 36, wobei der Kit ferner einen dritten Behälter einschließt, der eine Säure- oder Basen-Stopplösung enthält.

39. Verwendung nach Anspruch 35, wobei das chromogene Substrat aus der Gruppe ausgewählt ist, bestehend aus ortho-Phenylendiamin (OPD), 2, 2'-Azinobis-(3-ethylbenzothiazolin-6-sulfonsäure) (ABTS), Diaminobenzidin (DAB), 3, 3'-Dimethyloxybenzidin (ortho-Dianisidin oder ODN) und 3, 3' ,5, 5'-Tetramethylbenzidin (TMB).

40. Verwendung nach Anspruch 35, wobei der pH-Indikatorfarbstoff aus der Gruppe ausgewählt ist, bestehend aus Cresolrot, m-Cresolpurpur, Metanilgelb, 4-Phenylazodiphenylamin, Malachitgrün, Chinaldinrot, Orange IV, 2, 2', 2", 4, 4"-Pentamethoxytriphenylcarbinol und Kombinationen davon.

41. Verwendung nach Anspruch 35, wobei es sich bei dem chromogenen Substrat um 5-Aminosalicylsäure (5AS) handelt.

42. Verwendung nach Anspruch 41, wobei der pH-Indikatorfarbstoff aus der Gruppe ausgewählt ist, bestehend aus Phenolphthalein, Thymolphthalein, Alizaringelb R, Indigocarmin und Kombinationen davon.

43. Verwendung nach Anspruch 35, wobei es sich bei dem pH-Indikatorfarbstoff um m-Cresolpurpur handelt und das chromogene Substrat aus der Gruppe ausgewählt ist, bestehend aus 3, 3', 5, 5'-Tetramethylbenzidin (TMB) und 2, 2'-Azinobis-(3-ethylbenzothiazolin-6-sulfonsäure) (ABTS).

44. Verwendung nach Anspruch 35, wobei es sich bei dem enzymgekoppelten Assay um einen enzymgekoppelten Immunoadsorptionsassay (ELISA) handelt.

45. Verwendung nach Anspruch 35, wobei das chromogene Substrat aus der Gruppe ausgewählt ist, bestehend aus ortho-Phenylendiamin (OPD), 2, 2'-Azinobis-(3-ethylbenzothiazolin-6-sulfonsäure) (ABTS), Diaminobenzidin (DAB) und 3, 3'-Dimethyloxybenzidin (ortho-Dianisidin oder ODN), das durch ein Enzym in einem Reaktionsgefäß für den Assay in eine erste Farbe umgewandelt wird.

46. Verwendung nach Anspruch 45, wobei es sich bei dem chromogenen Substrat um p-Nitrophenylphosphat handelt.

47. Verwendung nach Anspruch 46, wobei der pH-Indikatorfarbstoff aus der Gruppe ausgewählt ist, bestehend aus Alizaringelb R, Indigocarmin und Kombinationen davon.

48. Verwendung einer Zusammensetzung zur Verwendung in einem enzymgekoppelten Immunoadsorptionsassay (ELISA) und zum Unterscheiden einer negativen Reaktion von einer falsch negativen Reaktion in dem ELISA, welche in einem Gemisch umfasst:
(a) ein chromogenes Substrat, das durch ein Peroxid oxidierbar ist, welches in dem ELISA in einem Reaktionsgemisch erzeugt wird, zu dem, um die Reaktion zu beenden, dann eine Stopplösung gegeben wird, um eine erste Farbe mit einer Absorption bei einer ersten Wellenlänge zu bilden; und
(b) einen pH-Indikatorfarbstoff, der farblos ist oder eine Farbe aufweist, die im Wesentlichen nicht mit dem Auge nachweisbar ist, wenn die Zusammensetzung auf ein Reaktionsgefäß für den ELISA angewendet wird, der aber eine zweite Farbe, die mit dem Auge nachweisbar ist und die eine Absorption bei einer zweiten Wellenlänge aufweist, produziert, wenn eine Säure- oder Basen-Stopplösung zu der Zusammensetzung in dem Reaktionsgefäß gegeben wird, wobei die Absorptionen bei der ersten und zweiten Wellenlänge spektrographisch gemessen werden, wobei Absorption bei der ersten Wellenlänge den Nachweis des Analyten anzeigt, Absorption bei der zweiten Wellenlänge eine negative Reaktion anzeigt und eine Abwesenheit von Absorption bei der ersten und zweiten Wellenlänge eine falsch negative Reaktion anzeigt, oder Nachweisen der zweiten Farbe mit dem Auge, wobei die zweite Farbe anzeigt, dass das Reaktionsgemisch die flüssige Lösung und die Stopplösung enthält.

49. Verwendung nach Anspruch 48, wobei das chromogene Substrat aus der Gruppe ausgewählt ist, bestehend aus ortho-Phenylendiamin (OPD), 2, 2'-Azinobis-(3-ethylbenzothiazolin-6-sulfonsäure) (ABTS), Diaminobenzidin (DAB) und 3, 3'-Dimethyloxybenzidin (ortho-Dianisidin oder ODN).

50. Verwendung nach Anspruch 49, wobei der pH-Indikatorfarbstoff aus der Gruppe ausgewählt ist, bestehend aus Cresolrot, m-Cresolpurpur, Metanilgelb, 4-Phenylazodiphenylamin, Malachitgrün, Chinaldinrot, Orange IV, 2, 2', 2", 4, 4"-Pentamethoxytriphenylcarbinol und Kombinationen davon.

51. Verwendung nach Anspruch 48, wobei es sich bei dem chromogenen Substrat um 5-Aminosalicylsäure (5AS) handelt.

52. Verwendung nach Anspruch 51, wobei der pH-Indikatorfarbstoff aus der Gruppe ausgewählt ist, bestehend aus Phenolphthalein, Thymolphthalein, Alizaringelb R, Indigocarmin und Kombinationen davon.

53. Verwendung nach Anspruch 48, wobei es sich bei dem pH-Indikatorfarbstoff um m-Cresolpurpur und bei dem chromogenen Substrat um 2, 2'-Azinobis-(3-ethylbenzothiazolin-6-sulfonsäure) (ABTS) handelt.

54. Verwendung nach Anspruch 48, wobei es sich bei dem chromogenen Substrat um 3, 3', 5, 5'-Tetramethylbenzidin (TMB) handelt.

55. Verwendung nach Anspruch 54, wobei es sich bei dem pH-Indikatorfarbstoff um m-Cresolpurpur handelt

56. Verfahren zum Nachweisen eines Analyten in einem enzymgekoppelten Assay und Unterscheiden einer negativen Reaktion von einer falsch negativen Reaktion in dem Assay, welches umfasst:
(a) Bereitstellen einer Zusammensetzung, umfassend ein Gemisch aus einem chromogenen Substrat, ausgewählt aus der Gruppe, bestehend aus ortho-Phenylendiamin (OPD), 2, 2'-Azinobis-(3-ethylbenzothiazolin-6-sulfonsäure) (ABTS), Diaminobenzidin (DAB) und 3, 3'-Dimethyloxybenzidin (ortho-Dianisidin oder ODN), das durch ein Enzym in einem Reaktionsgefäß für den Assay zu einer ersten Farbe umgewandelt wird, die eine Absorption bei einer ersten Wellenlänge aufweist, und einem pH-Indikatorfarbstoff, der eine zweite mit dem Auge nachweisbar Farbe produziert und eine Absorption bei der zweiten Wellenlänge aufweist, wenn eine Säure- oder Basen-Stopplösung zu der Zusammensetzung in dem Reaktionsgefäß gegeben wird;
(b) Zugeben eines Aliquots der Zusammensetzung zu dem Reaktionsgefäß für den enzymgekoppelten Assay, um ein Reaktionsgemisch zu bilden;
(c) Inkubieren des Reaktionsgemisches über eine Zeit hinweg, die ausreicht die erste Farbe zu bilden;
(d) Zugeben der Stopplösung zu dem Reaktionsgemisch, um die Reaktion zu beenden und die zweite Farbe mit der zweiten Wellenlänge zu erzeugen; und
(e) spektrographisches Messen der Absorptionen bei der ersten und zweiten Wellenlänge, wobei Absorption bei der ersten Wellenlänge den Nachweis des Analyten anzeigt, Absorption nur bei der zweiten Wellenlänge die negative Reaktion anzeigt und Abwesenheit von Absorption bei der ersten und zweiten Wellenlänge die falsch negative Reaktion anzeigt, oder Nachweisen der zweiten Farbe mit dem Auge, wobei die zweite Farbe anzeigt, dass das Reaktionsgemisch die Zusammensetzung und die Stopplösung enthält.

57. Verfahren nach Anspruch 56, wobei es sich bei dem Enzym um eine alkalische Phosphatase, bei dem chromogenen Substrat um p-Nitrophenylphosphat und bei der Stopplösung um eine Base handelt.

58. Verfahren nach Anspruch 57, wobei der pH-Indikatorfarbstoff aus der Gruppe ausgewählt ist, bestehend aus Alizaringelb R, Indigocarmin und Kombinationen davon.

59. Verfahren nach Anspruch 56, wobei es sich bei dem Assay um einen enzymgekoppelten Immunoadsorptionsassay (ELISA) handelt.

60. Verfahren nach Anspruch 56 oder 59, wobei der Assay aus der Gruppe ausgewählt ist, bestehend aus direktem, indirektem und kompetitivem Assay.

61. Verfahren nach Anspruch 60, wobei der pH-Indikatorfarbstoff aus der Gruppe ausgewählt ist, bestehend aus Cresolrot, m-Cresolpurpur, Metanilgelb, 4-Phenylazodiphenylamin, Malachitgrün, Orange IV, 2, 2', 2", 4, 4"-Pentamethoxytriphenylcarbinol und Kombinationen davon.

62. Verwendung einer Zusammensetzung zur Verwendung in einem enzymgekoppelten Assay und zum Unterscheiden einer negativen Reaktion von einer falsch negativen Reaktion in dem Assay, die in einem Gemisch umfasst:
(a) ein chromogenes Substrat ist ausgewählt aus der Gruppe, bestehend aus ortho-Phenylendiamin (OPD), 2, 2'-Azinobis-(3-ethylbenzothiazolin-6-sulfonsäure) (ABTS), Diaminobenzidin (DAB) und 3, 3'-Dimethyloxybenzidin (ortho-Dianisidin oder ODN), das durch ein Enzym in einem Reaktionsgefäß für den Assay in eine erste Farbe mit einer Absorption bei einer ersten Wellenlänge umgewandelt wird; und
(b) einen pH-Indikatorfarbstoff, der eine zweite mit dem Auge nachweisbare Farbe produziert und eine Absorption bei einer zweiten Wellenlänge aufweist, wenn eine Säure- oder Basen-Stopplösung zu der Zusammensetzung in dem Reaktionsgefäß gegeben wird; wobei die Absorptionen bei der ersten und zweiten Wellenlänge spektrographisch gemessen werden, wobei Absorption bei der ersten Wellenlänge den Nachweis des Analyten anzeigt, Absorption bei der zweiten Wellenlänge eine negative Reaktion anzeigt und eine Abwesenheit von Absorption bei der ersten und zweiten Wellenlänge eine falsch negative Reaktion anzeigt, oder Nachweisen der zweiten Farbe mit dem Auge, wobei die zweite Farbe anzeigt, dass das Reaktionsgemisch die flüssige Lösung und die Stopplösung enthält.

63. Verwendung nach Anspruch 62, wobei es sich bei dem chromogenen Substrat um p-Nitrophenylphosphat handelt.

64. Verwendung nach Anspruch 63, wobei der pH-Indikatorfarbstoff aus der Gruppe ausgewählt ist, bestehend aus Alizaringelb R, Indigocarmin und Kombinationen davon.

65. Verwendung nach Anspruch 62, wobei der pH-Indikatorfarbstoff aus der Gruppe ausgewählt ist, bestehend aus Cresolrot, m-Cresolpurpur, Metanilgelb, 4-Phenylazodiphenylamin, Malachitgrün, Orange IV, 2, 2', 2", 4, 4"-Pentamethoxytriphenylcarbinol und Kombinationen davon.

66. Verwendung eines Enzymimmunoassays zum Unterscheiden einer negativen Reaktion von einer falsch negativen Reaktion in dem Immunoassay und wobei der Immunoassay zum colorimetrischen Nachweis eines Analyten bestimmt ist, derart, dass eine Menge eines ersten Antikörpers an eine feste Unterlage in einem Reaktionsgefäß adsorbiert wird; ein Konjugat zwischen einem immunologischen Reagenz und einer Peroxidase gebildet wird; das Konjugat mit einer auf den Analyten zu testenden Probe gemischt wird, der Analyt an den ersten Antikörper und an das Konjugat bindet, um einen immunologischen Komplex in fester Phase zu bilden; und die Menge des Analyten durch Messen der Reaktion des immunologischen Komplexes mit einem durch Peroxid oxidierbaren chromogenen Substrat und der Peroxidase bestimmt wird, umfassend:
(a) Bereitstellen einer flüssigen Lösung, umfassend das chromogene Substrat, Peroxid und einen pH-Indikatorfarbstoff, wobei es sich bei dem chromogenen Substrat um TMB oder OPD handelt und dieses zu einer ersten Farbe mit einer Absorption bei einer ersten Wellenlänge oxidierbar ist, wenn ein Reaktionsgemisch zwischen der flüssigen Lösung und dem immunologischen Komplex gebildet wird, und es weiter von der ersten Farbe zu einer zweiten Farbe mit einer Absorption bei einer zweiten Wellenlänge oxidierbar ist, wenn eine Stopplösung zu dem Reaktionsgefäß gegeben wird, und der pH-Indikatorfarbstoff eine dritte Farbe produziert, die mit dem Auge nachweisbar ist und die eine Absorption bei einer dritten Wellenlänge aufweist, wenn eine Stopplösung zu dem Reaktionsgefäß gegeben wird, welches die flüssige Lösung enthält;
(b) Mischen der flüssigen Lösung mit dem immunologischen Komplex in dem Reaktionsgefäß, um ein Reaktionsgemisch zu bilden, welches das chromogene Substrat zu der ersten Farbe oxidiert;
(c) Zugeben der Stopplösung zu dem Reaktionsgemisch in dem Reaktionsgefäß, um die zweite und dritte Farbe zu produzieren; und
(d) spektrographisches Messen der Absorptionen bei der ersten, zweiten und dritten Wellenlänge, wobei eine positive Reaktion durch Absorption bei der zweiten Wellenlänge nachgewiesen wird, Reaktionen, die keine Absorption bei der ersten oder zweiten Wellenlänge aufweisen, aber Absorption bei der dritten Wellenlänge aufweisen, als negativ gewertet werden und wobei alle Reaktionen ohne Absorption bei der dritten Wellenlänge als falsch negativ gewertet werden.

67. Verwendung eines Enzymimmunoassays zum Unterscheiden einer negativen Reaktion von einer falsch negativen Reaktion in dem Immunoassay und wobei der Immunoassay zum colorimetrischen Nachweis eines Antikörpers bestimmt ist, derart, dass eine Menge eines Analyten an eine feste Unterlage in einem Reaktionsgefäß adsorbiert wird; ein Konjugat zwischen einem immunologischen Reagenz und einer Peroxidase gebildet wird; das Konjugat mit einer auf den Antikörper zu testenden Probe gemischt wird, der Antikörper an den Analyten und an das Konjugat bindet, um einen immunologischen Komplex in fester Phase zu bilden; und die Menge des Antikörpers durch Messen der Reaktion des immunologischen Komplexes mit einem durch Peroxid oxidierbaren chromogenen Substrat und der Peroxidase bestimmt wird, umfassend:
(a) Bereitstellen einer flüssigen Lösung, umfassend das chromogene Substrat, Peroxid und einen pH-Indikatorfarbstoff, wobei es sich bei dem chromogenen Substrat um TMB oder OPD handelt und dieses zu einer ersten Farbe mit einer Absorption bei einer ersten Wellenlänge oxidierbar ist, wenn ein Reaktionsgemisch zwischen der flüssigen Lösung und dem immunologischen Komplex gebildet wird und es weiter von der ersten Farbe zu einer zweiten Farbe mit einer Absorption bei einer zweiten Wellenlänge oxidierbar ist, wenn eine Stopplösung zu dem Reaktionsgefäß gegeben wird, und der pH-Indikatorfarbstoff eine dritte Farbe produziert, die mit dem Auge nachweisbar ist und die eine Absorption bei einer dritten Wellenlänge aufweist, wenn eine Stopplösung zu dem Reaktionsgefäß gegeben wird, welches die flüssige Lösung enthält;
(b) Mischen der flüssigen Lösung mit dem immunologischen Komplex in dem Reaktionsgefäß, um ein Reaktionsgemisch zu bilden, welches das chromogene Substrat zu der ersten Farbe oxidiert;
(c) Zugeben der Stopplösung zu dem Reaktionsgemisch in dem Reaktionsgefäß, um die zweite und dritte Farbe zu produzieren; und
(d) spektrographisches Messen der Absorption bei der ersten, zweiten und dritten Wellenlänge, wobei eine positive Reaktion durch Absorption bei der zweiten Wellenlänge nachgewiesen wird, Reaktionen, die keine Absorption bei der ersten oder zweiten Wellenlänge aufweisen, aber Absorption bei der dritten Wellenlänge aufweisen, als negativ gewertet werden und wobei alle Reaktionen ohne Absorption bei der dritten Wellenlänge als falsch negativ gewertet werden.

## Revendications

1. Procédé destiné à détecter un analyte dans un dosage par méthode immunoenzymatique (ELISA) utilisant une peroxydase et à distinguer une réaction négative d'une fausse réaction négative dans l'ELISA, comprenant les étapes consistant à :
(a) fournir une composition comprenant un mélange d'un substrat chromogène qui est oxydable par un peroxyde dans une réaction destinée au dosage par ELISA catalysée par la peroxydase de l'ELISA afin de former un premier produit coloré présentant une absorbance à une première longueur d'onde, un colorant indicateur de pH qui forme un second produit coloré qui est détectable à l'oeil et qui présente une absorbance à une seconde longueur d'onde lorsqu'une solution d'arrêt est ajoutée à la composition dans un récipient de réaction, et un peroxyde ;
(b) ajouter une partie aliquote de la composition à chacun des récipients de réaction destinés au dosage par ELISA afin de former un mélange réactionnel ;
(c) incuber le mélange réactionnel pendant un temps suffisant pour oxyder le substrat chromogène en le premier produit coloré ;
(d) ajouter la solution d'arrêt au mélange réactionnel pour arrêter la réaction et pour générer le second produit coloré ; et
(e) mesurer les absorbances par le biais d'analyses spectrographiques à la première et à la seconde longueur d'onde, une absorbance à la première longueur d'onde indiquant la détection de l'analyte, une absorbance uniquement à la seconde longueur d'onde indiquant la réaction négative, et une absence d'absorbance à la première et à la seconde longueurs d'onde indiquant la fausse réponse négative ou détecter la seconde couleur à l'oeil, la seconde couleur indiquant que le mélange réactionnel contient la composition et la solution d'arrêt.

2. Procédé selon la revendication 1, dans lequel l'indicateur de pH est incolore ou a une couleur qui n'est pratiquement pas détectable à l'oeil lorsque la composition est ajoutée dans un récipient de réaction destiné à l'ELISA mais qui forme le second produit coloré qui est détectable à l'oeil et qui présente l'absorbance à la seconde longueur d'onde lorsque la solution d'arrêt est ajoutée à la composition dans le récipient de réaction.

3. Procédé selon la revendication 1, dans lequel la solution d'arrêt est un acide et le substrat chromogène est choisi dans le groupe comprenant l'orthophénylènediamine (OPD), l'acide 2,2'-azinobis-(3-éthyl-benzothiazoline-6-sulfonique) (ABTS), la diaminobenzidine (DAB), la 3,3'-diméthyloxybenzidine (l'ortho-dianisidine ou ODN), et la 3,3',5,5'-tétraméthylbenzidine (TMB).

4. Procédé selon la revendication 1, dans lequel la solution d'arrêt est un acide et le substrat chromogène est l'acide 2,2'-azinobis-(3-éthyl-benzothiazoline-6-sulfonique) (ABTS).

5. Procédé selon la revendication 1, dans lequel le colorant indicateur de pH est choisi dans le groupe comprenant le rouge de crésol, le pourpre de m-crésol, le jaune métanile, la 4-phénylazodiphénylamine, le vert malachite, le colorant orange IV, le 2,2',2",4,4"-pentaméthoxytriphényl carbinol, et des combinaisons de ceux-ci.

6. Procédé selon la revendication 1, dans lequel la solution d'arrêt est une base et le substrat chromogène est l'acide 5-aminosalicylique (5AS).

7. Procédé selon la revendication 6, dans lequel le colorant indicateur de pH est choisi dans le groupe comprenant la phénolphtaléine, la thymolphtaléïne, le jaune d'alizarine R, le carmin d'indigo, et des combinaisons de ceux-ci.

8. Procédé selon la revendication 1, dans lequel l'ELISA est choisi dans le groupe comprenant des ELISA directe, indirecte, et compétitive.

9. Procédé selon la revendication 1, dans lequel le substrat chromogène est la 3,3',5,5'-tétraméthylbenzidine (TMB).

10. Procédé selon la revendication 9, dans lequel le colorant indicateur de pH est le pourpre de m-crésol.

11. Procédé selon la revendication 9, dans lequel la première longueur d'onde est d'environ 450 nm et la seconde longueur d'onde est supérieure à 500 nm.

12. Procédé selon la revendication 3 ou 4, dans lequel l'acide est choisi dans le groupe comprenant HCl, H₂SO₄, et H₂PO₄.

13. Utilisation d'un dosage par méthode immunoenzymatique pour distinguer une réaction négative d'une fausse réaction négative dans le dosage par méthode immunoenzymatique et dans laquelle le dosage par méthode immunoenzymatique est destiné à détecter de manière colorimétrique un analyte d'un certain type, dans lequel une quantité d'un premier anticorps est adsorbée sur un support solide dans un récipient de réaction ; un conjugué est formé entre un réactif immunologique et une peroxydase ; le conjugué est mélangé à un échantillon à tester à la recherche de l'analyte, l'analyte se lie au premier anticorps et au conjugué afin de former un complexe immunologique en phase solide ; et la quantité de l'analyte est déterminée en mesurant la réaction du complexe immunologique avec un substrat chromogène oxydable par un peroxyde et la peroxydase, comprenant les étapes consistant à :
(a) fournir une solution liquide comprenant le substrat chromogène, le peroxyde, et un colorant indicateur de pH, dans laquelle le substrat chromogène est oxydable en un premier produit coloré présentant une absorbance à une première longueur d'onde et le colorant indicateur de pH produit un second produit coloré qui est détectable à l'oeil et qui présente une absorbance à une seconde longueur d'onde lorsqu'une solution d'arrêt est ajoutée dans le récipient de réaction contenant la solution liquide ;
(b) mélanger la solution liquide au complexe immunologique dans le récipient de réaction afin de former un mélange réactionnel qui oxyde le substrat chromogène pour former le premier produit coloré ;
(c) ajouter la solution d'arrêt au mélange réactionnel dans le récipient de réaction pour produire le second produit coloré et,
(d) mesurer les absorbances par le biais d'analyses spectrographiques à la première et à la seconde longueurs d'onde, une absorbance à la première longueur d'onde indiquant la détection de l'analyte, une absorbance à la seconde longueur d'onde indiquant une réaction négative, et une absence d'absorbance à la première et à la seconde longueur d'onde indiquant une fausse réaction négative ou détecter la seconde couleur à l'oeil, la seconde couleur indiquant que le mélange réactionnel contient la solution liquide et la solution d'arrêt.

14. Utilisation selon la revendication 13, dans laquelle l'indicateur de pH est incolore ou a une couleur qui n'est pratiquement pas détectable à l'oeil lorsque la composition est ajoutée dans un récipient de réaction destiné à l'ELISA mais qui forme le second produit coloré qui est détectable à l'oeil et qui présente l'absorbance à la seconde longueur d'onde lorsque la solution d'arrêt est ajoutée à la composition dans le récipient de réaction.

15. Utilisation selon la revendication 13, dans laquelle la solution d'arrêt est un acide et le substrat chromogène est choisi dans le groupe comprenant l'orthophénylènediamine (OPD), l'acide 2,2'-azinobis-(3-éthyl-benzothiazoline-6-sulfonique) (ABTS), la diaminobenzidine (DAB), la 3,3'-diméthyloxybenzidine (l'ortho-dianisidine ou ODN), et la 3,3',5,5'-tétraméthylbenzidine (TMB).

16. Utilisation selon la revendication 13, dans laquelle la solution d'arrêt est un acide et le substrat chromogène est l'acide 2,2'-azinobis-(3-éthyl-benzothiazoline-6-sulfonique) (ABTS).

17. Utilisation selon la revendication 13, dans laquelle le colorant indicateur de pH est choisi dans le groupe comprenant le rouge de crésol, le pourpre de m-crésol, le jaune métanile, la 4-phénylazodiphénylamine, le vert malachite, le colorant orange IV, le 2,2',2",4,4"-pentaméthoxytriphényl carbinol, et des combinaisons de ceux-ci.

18. Utilisation selon la revendication 13, dans laquelle la solution d'arrêt est une base et le substrat chromogène est l'acide 5-aminosalicylique (5AS).

19. Utilisation selon la revendication 18, dans laquelle le colorant indicateur de pH est choisi dans le groupe comprenant la phénolphtaléine, la thymolphtaléïne, le jaune d'alizarine R, le carmin d'indigo, et des combinaisons de ceux-ci.

20. Utilisation selon la revendication 13, dans laquelle le substrat chromogène est la 3,3',5,5'-tétraméthylbenzidine (TMB).

21. Utilisation selon la revendication 20, dans laquelle le colorant indicateur de pH est le pourpre de m-crésol.

22. Utilisation selon la revendication 20, dans laquelle la première longueur d'onde est d'environ 450 nm et la seconde longueur d'onde est supérieure à 500 nm.

23. Utilisation selon la revendication 15 ou 16, dans laquelle l'acide est choisi dans le groupe comprenant HCl, H₂SO₄, et H₂PO₄.

24. Utilisation d'un dosage par méthode immunoenzymatique pour distinguer une réaction négative d'une fausse réaction négative dans le dosage par méthode immunoenzymatique et dans laquelle le dosage par méthode immunoenzymatique est destiné à détecter de manière colorimétrique un anticorps d'un certain type, dans lequel une quantité d'un analyte est adsorbée sur un support solide dans un récipient de réaction ; un conjugué est formé entre un réactif immunologique et une peroxydase ; le conjugué est mélangé à un échantillon à tester à la recherche de l'anticorps, l'anticorps se lie à l'analyte et au conjugué afin de former un complexe immunologique en phase solide ; et la quantité de l'anticorps est déterminée en mesurant la réaction du complexe immunologique avec un substrat chromogène oxydable par un peroxyde et la peroxydase, comprenant les étapes consistant à :
(a) fournir une solution liquide comprenant le substrat chromogène, le peroxyde, et un colorant indicateur de pH, dans laquelle le substrat chromogène est oxydable en un premier produit coloré présentant une absorbance à une première longueur d'onde et le colorant indicateur de pH produit un second produit coloré qui est détectable à l'oeil et qui présente une absorbance à une seconde longueur d'onde lorsqu'une solution d'arrêt est ajoutée dans le récipient de réaction contenant la solution liquide ;
(b) mélanger la solution liquide au complexe immunologique dans le récipient de réaction afin de former un mélange réactionnel qui oxyde le substrat chromogène pour former le premier produit coloré ;
(c) ajouter la solution d'arrêt au mélange réactionnel dans le récipient de réaction pour produire le second produit coloré ; et,
(d) mesurer les absorbances par le biais d'analyses spectrographiques à la première et à la seconde longueurs d'onde, une absorbance à la première longueur d'onde indiquant la détection de l'analyte, une absorbance à la seconde longueur d'onde indiquant une réaction négative, et une absence d'absorbance à la première et à la seconde longueur d'onde indiquant une fausse réaction négative ou détecter la seconde couleur à l'oeil, la seconde couleur indiquant que le mélange réactionnel contient la solution liquide et la solution d'arrêt.

25. Utilisation selon la revendication 24, dans laquelle l'indicateur de pH est incolore ou a une couleur qui n'est pratiquement pas détectable à l'oeil lorsque la composition est ajoutée dans un récipient de réaction destiné à l'ELISA mais qui forme le second produit coloré qui est détectable à l'oeil et qui présente l'absorbance à la seconde longueur d'onde lorsque la solution d'arrêt est ajoutée à la composition dans le récipient de réaction.

26. Utilisation selon la revendication 24, dans laquelle la solution d'arrêt est un acide et le substrat chromogène est choisi dans le groupe comprenant l'orthophénylènediamine (OPD), l'acide 2,2'-azinobis-(3-éthyl-benzothiazoline-6-sulfonique) (ABTS), la diaminobenzidine (DAB), la 3,3'-diméthyloxybenzidine (l'ortho-dianisidine ou ODN), et la 3,3',5,5'-tétraméthylbenzidine (TMB).

27. Utilisation selon la revendication 24, dans laquelle la solution d'arrêt est un acide et le substrat chromogène est l'acide 2,2'-azinobis-(3-éthyl-benzothiazoline-6-sulfonique) (ABTS).

28. Utilisation selon la revendication 24, dans laquelle le colorant indicateur de pH est choisi dans le groupe comprenant le rouge de crésol, le pourpre de m-crésol, le jaune métanile, la 4-phénylazodiphénylamine, le vert malachite, le colorant orange IV, le 2,2',2",4,4"-pentaméthoxytriphényl carbinol, et des combinaisons de ceux-ci.

29. Utilisation selon la revendication 24, dans laquelle la solution d'arrêt est une base et le substrat chromogène est l'acide 5-aminosalicylique (5AS).

30. Utilisation selon la revendication 24, dans laquelle le colorant indicateur de pH est choisi dans le groupe comprenant la phénolphtaléine, la thymolphtaléïne, le jaune d'alizarine R, le carmin d'indigo, et des combinaisons de ceux-ci.

31. Utilisation d'un dosage par méthode immunoenzymatique selon la revendication 24, dans laquelle le substrat chromogène est la 3,3',5,5'-tétraméthylbenzidine (TMB).

32. Utilisation selon la revendication 31, dans laquelle le colorant indicateur de pH est le pourpre de m-crésol.

33. Utilisation selon la revendication 31, dans laquelle la première longueur d'onde est d'environ 450 nm et la seconde longueur d'onde est supérieure à 500 nm.

34. Utilisation selon la revendication 31, dans laquelle l'acide est choisi dans le groupe comprenant HCl, H₂SO₄, et H₂PO₄.

35. Utilisation d'un kit destiné à détecter un analyte dans un dosage par méthode enzymatique et à distinguer une réaction négative d'une fausse réaction négative dans le dosage, comprenant :
un ou plusieurs premiers conteneurs contenant chacun un mélange d'un substrat chromogène présentant une absorbance à une première longueur
d'onde, d'un colorant indicateur de pH approprié pour une utilisation avec le substrat chromogène, qui produit un produit coloré détectable à l'oeil qui présente une absorbance à une seconde longueur d'onde lorsqu'une solution d'arrêt acide ou basique est ajoutée au mélange dans le récipient de réaction, et d'un peroxyde,
dans laquelle les absorbances sont mesurées par le biais d'analyses spectrographiques à la première et à la seconde longueur d'onde,
une absorbance à la première longueur d'onde indiquant la détection de l'analyte, une absorbance à la seconde longueur d'onde indiquant une réaction négative, et une absence d'absorbance à la première et à la seconde longueur d'onde indiquant une fausse réaction négative ou une détection de la seconde couleur à l'oeil, la seconde couleur indiquant que le mélange réactionnel contient la solution liquide et la solution d'arrêt.

36. Utilisation selon la revendication 35, dans laquelle l'indicateur de pH est incolore ou a une couleur qui n'est pratiquement pas détectable à l'oeil lorsque la composition est ajoutée dans un récipient de réaction destiné au dosage mais qui forme le second produit coloré qui est détectable à l'oeil et qui présente l'absorbance à la seconde longueur d'onde lorsque la solution d'arrêt est ajoutée à la composition dans le récipient de réaction.

37. Utilisation selon la revendication 35, dans laquelle le premier conteneur contient le substrat chromogène et le colorant indicateur de pH et le second conteneur contient le peroxyde.

38. Utilisation selon la revendication 35 ou 36, dans laquelle le kit comprend en outre un troisième conteneur contenant une solution d'arrêt acide ou basique.

39. Utilisation selon la revendication 35, dans laquelle le substrat chromogène est choisi dans le groupe comprenant l'orthophénylènediamine (OPD), l'acide 2,2'-azinobis-(3-éthyl-benzothiazoline-6-sulfonique) (ABTS), la diaminobenzidine (DAB), la 3,3'-diméthyloxybenzidine (l'ortho-dianisidine ou ODN), et la 3,3',5,5'-tétraméthylbenzidine (TMB).

40. Utilisation selon la revendication 35, dans laquelle le colorant indicateur de pH est choisi dans le groupe comprenant le rouge de crésol, le pourpre de m-crésol, le jaune métanile, la 4-phénylazodiphénylamine, le vert malachite, le rouge de quinaldine, le colorant orange IV, le 2,2',2",4,4"-pentaméthoxytriphényl carbinol, et des combinaisons de ceux-ci.

41. Utilisation selon la revendication 35, dans laquelle le substrat chromogène est l'acide 5-aminosalicylique (5AS).

42. Utilisation selon la revendication 41, dans laquelle le colorant indicateur de pH est choisi dans le groupe comprenant la phénolphtaléine, la thymolphtaléïne, le jaune d'alizarine R, le carmin d'indigo, et des combinaisons de ceux-ci.

43. Utilisation selon la revendication 35, dans laquelle le colorant indicateur de pH est le pourpre de m-crésol et le substrat chromogène est choisi dans le groupe comprenant la 3,3',5,5'-tétraméthylbenzidine (TMB) et l'acide 2,2'-azinobis-(3-éthyl-benzothiazoline-6-sulfonique) (ABTS).

44. Utilisation selon la revendication 35, dans laquelle le dosage par méthode enzymatique est un dosage par méthode immunoenzymatique (ELISA).

45. Utilisation selon la revendication 35, dans laquelle le substrat chromogène est choisi dans le groupe comprenant l'orthophénylènediamine (OPD), l'acide 2,2'-azinobis-(3-éthyl-benzothiazoline-6-sulfonique) (ABTS), la diaminobenzidine (DAB), et la 3,3'-diméthyloxybenzidine (l'ortho-dianisidine ou ODN) qui est converti en un premier produit coloré par une enzyme dans un récipient de réaction destiné au dosage.

46. Utilisation selon la revendication 45, dans laquelle le substrat chromogène est le phosphate de p-nitrophényle.

47. Utilisation selon la revendication 46, dans laquelle le colorant indicateur de pH est choisi dans le groupe comprenant le jaune d'alizarine R, le carmin d'indigo, et des combinaisons de ceux-ci.

48. Utilisation d'une composition destinée à être utilisée dans un dosage par méthode immunoenzymatique (ELISA) et à distinguer une réaction négative d'une fausse réaction négative dans l'ELISA, qui comprend sous forme de mélange :
(a) un substrat chromogène qui est oxydable par un peroxyde généré dans l'ELISA dans un mélange réactionnel auquel est ensuite ajoutée une solution d'arrêt pour arrêter la réaction et former un premier produit coloré présentant une absorbance à une première longueur d'onde ; et
(b) un colorant indicateur de pH qui est incolore ou qui a une couleur qui n'est pratiquement pas détectable à l'oeil lorsque la composition est ajoutée dans un récipient de réaction destiné à l'ELISA mais qui produit un second produit coloré détectable à l'oeil et qui présente un absorbance à une seconde longueur d'onde lorsqu'une solution d'arrêt acide ou basique est ajoutée à la composition dans le récipient de réaction, les absorbances étant mesurées par le biais d'analyses spectrographiques à la première et à la seconde longueur d'onde, une absorbance à la première longueur d'onde indiquant la détection de l'analyte, une absorbance à la seconde longueur d'onde indiquant une réaction négative, et une absence d'absorbance à la première et à la seconde longueur d'onde indiquant une fausse réaction négative ou la seconde couleur étant détectée à l'oeil, la seconde couleur indiquant que le mélange réactionnel contient la solution liquide et la solution d'arrêt.

49. Utilisation selon la revendication 48, dans laquelle le substrat chromogène est choisi dans le groupe comprenant l'orthophénylènediamine (OPD), l'acide 2,2'-azinobis-(3-éthyl-benzothiazoline-6-sulfonique) (ABTS), la diaminobenzidine (DAB), et la 3,3'-diméthyloxybenzidine (l'ortho-dianisidine ou ODN).

50. Utilisation selon la revendication 49, dans laquelle le colorant indicateur de pH est choisi dans le groupe comprenant le rouge de crésol, le pourpre de m-crésol, le jaune métanile, la 4-phénylazodiphénylamine, le vert malachite, le rouge de quinaldine, le colorant orange IV, le 2,2',2",4,4"-pentaméthoxytriphényl carbinol, et des combinaisons de ceux-ci.

51. Utilisation selon la revendication 48, dans laquelle le substrat chromogène est l'acide 5-aminosalicylique (5AS).

52. Utilisation selon la revendication 51, dans laquelle le colorant indicateur de pH est choisi dans le groupe comprenant la phénolphtaléine, la thymolphtaléïne, le jaune d'alizarine R, le carmin d'indigo, et des combinaisons de ceux-ci.

53. Utilisation selon la revendication 48, dans laquelle le colorant indicateur de pH est le pourpre de m-crésol et le substrat chromogène est l'acide 2,2'-azinobis-(3-éthyl-benzothiazoline-6-sulfonique) (ABTS).

54. Utilisation selon la revendication 48, dans laquelle le substrat chromogène est la 3,3',5,5'-tétraméthylbenzidine (TMB).

55. Utilisation selon la revendication 54, dans laquelle le colorant indicateur de pH est le pourpre de m-crésol.

56. Procédé destiné à détecter un analyte dans un dosage par méthode enzymatique et à distinguer une réaction négative d'une fausse réaction négative dans le dosage, comprenant les étapes consistant à :
(a) fournir une composition comprenant un mélange d'un substrat chromogène choisi dans le groupe comprenant l'orthophénylènediamine (OPD), l'acide 2,2'-azinobis-(3-éthyl-benzothiazoline-6-sulfonique) (ABTS), la diaminobenzidine (DAB), et la 3,3'-diméthyloxybenzidine (l'ortho-dianisidine ou ODN), qui est converti par une enzyme dans un récipient de réaction destiné à l'ELISA afin de former un premier produit coloré qui présente une absorbance à une première longueur d'onde et un colorant indicateur de pH qui produit un second produit coloré détectable à l'oeil et qui présente une absorbance à une seconde longueur d'onde lorsqu'une solution d'arrêt acide ou basique est ajoutée à la composition dans le récipient de réaction ;
(b) ajouter une partie aliquote de la composition dans le récipient de réaction destiné au dosage par méthode enzymatique afin de former un mélange réactionnel ;
(c) incuber le mélange réactionnel pendant un temps suffisant pour produire le premier produit coloré ;
(d) ajouter la solution d'arrêt au mélange réactionnel pour arrêter la réaction et pour générer le second produit coloré présentant une absorbance à la seconde longueur d'onde ; et
(e) mesurer les absorbances par le biais d'analyses spectrographiques à la première et à la seconde longueur d'onde,
une absorbance à la première longueur d'onde indiquant la détection de l'analyte,
une absorbance uniquement à la seconde longueur d'onde indiquant la réaction négative, et une absence d'absorbance à la première et à la seconde longueur d'onde indiquant la fausse réponse négative ou détecter la seconde couleur à l'oeil, la seconde couleur indiquant que le mélange réactionnel contient la composition et la solution d'arrêt.

57. Procédé selon la revendication 56, dans lequel l'enzyme est une phosphatase alcaline, le substrat chromogène est le phosphate de p-nitrophényle, et la solution d'arrêt est une base.

58. Procédé selon la revendication 57, dans lequel le colorant indicateur de pH est choisi dans le groupe comprenant le jaune d'alizarine R, le carmin d'indigo, et des combinaisons de ceux-ci.

59. Procédé selon la revendication 56, dans lequel le dosage est un dosage par méthode immunoenzymatique (ELISA).

60. Procédé selon la revendication 56 ou 59, dans lequel le dosage est choisi dans le groupe comprenant une ELISA directe, indirecte, et compétitive.

61. Procédé selon la revendication 60, dans lequel le colorant indicateur de pH est choisi dans le groupe comprenant le rouge de crésol, le pourpre de m-crésol, le jaune métanile, la 4-phénylazodiphénylamine, le vert malachite, le colorant orange IV, le 2,2',2",4,4"-pentaméthoxytriphényl carbinol, et des combinaisons de ceux-ci.

62. Utilisation d'une composition destinée à être utilisée dans un dosage par méthode enzymatique et pour distinguer une réaction négative d'une fausse réaction négative dans le dosage, qui comprend sous forme de mélange :
(a) un substrat chromogène qui est choisi dans le groupe comprenant l'orthophénylènediamine (OPD), l'acide 2,2'-azinobis-(3-éthyl-benzothiazoline-6-sulfonique) (ABTS), la diaminobenzidine (DAB), et la 3,3'-diméthyloxybenzidine (l'ortho-dianisidine ou ODN) qui est converti en un premier produit coloré présentant une absorbance à une première longueur d'onde par une enzyme dans un récipient de réaction destiné au dosage ; et
(b) un colorant indicateur de pH qui produit un second produit coloré détectable à l'oeil et qui présente une absorbance à une seconde longueur d'onde lorsqu'une solution d'arrêt acide ou basique est ajoutée à la composition dans le récipient de réaction ;
dans laquelle les absorbances sont mesurées par le biais d'analyses spectrographiques à la première et à la seconde longueur d'onde, une absorbance à la première longueur d'onde indiquant la détection de l'analyte, une absorbance à la seconde longueur d'onde indiquant une réaction négative, et une absence d'absorbance à la première et à la seconde longueur d'onde indiquant une fausse réaction négative ou dans laquelle la seconde couleur est détectée à l'oeil, la seconde couleur indiquant que le mélange réactionnel contient la solution liquide et la solution d'arrêt.

63. Utilisation selon la revendication 62, dans laquelle le substrat chromogène est le phosphate de p-nitrophényle.

64. Utilisation selon la revendication 63, dans laquelle le colorant indicateur de pH est choisi dans le groupe comprenant le jaune d'alizarine R, le carmin d'indigo, et des combinaisons de ceux-ci.

65. Utilisation selon la revendication 62, dans laquelle le colorant indicateur de pH est choisi dans le groupe comprenant le rouge de crésol, le pourpre de m-crésol, le jaune métanile, la 4-phénylazodiphénylamine, le vert malachite, le colorant orange IV, le 2,2',2",4,4"-pentaméthoxytriphényl carbinol, et des combinaisons de ceux-ci.

66. Utilisation d'un dosage par méthode immunoenzymatique pour distinguer une réaction négative d'une fausse réaction négative dans le dosage par méthode immunoenzymatique et dans laquelle le dosage par méthode immunoenzymatique est destiné à détecter de manière colorimétrique un analyte d'un certain type, dans lequel une quantité d'un premier anticorps est adsorbée sur un support solide dans un récipient de réaction ; un conjugué est formé entre un réactif immunologique et une peroxydase ; le conjugué est mélangé à un échantillon à tester à la recherche de l'analyte, l'analyte se lie au premier anticorps et au conjugué afin de former un complexe immunologique en phase solide ; et la quantité de l'analyte est déterminée en mesurant la réaction du complexe immunologique avec un substrat chromogène oxydable par un peroxyde et la peroxydase, comprenant les étapes consistant à :
(a) fournir une solution liquide comprenant le substrat chromogène, le peroxyde, et un colorant indicateur de pH, dans laquelle le substrat chromogène est la TMB ou l'OPD et est oxydable en un premier produit coloré présentant une absorbance à une première longueur d'onde lorsqu'un mélange réactionnel est formé entre la solution liquide et le complexe immunologique et est encore oxydable formant un deuxième produit coloré à partir du premier produit coloré présentant une absorbance à une deuxième longueur d'onde lorsqu'une solution d'arrêt est ajoutée dans le récipient de réaction et le colorant indicateur de pH produit un troisième produit coloré qui est détectable à l'oeil et qui présente une absorbance à une troisième longueur d'onde lorsqu'une solution d'arrêt est ajoutée dans le récipient de réaction contenant la solution liquide ;
(b) mélanger la solution liquide au complexe immunologique dans le récipient de réaction afin de former un mélange réactionnel qui oxyde le substrat chromogène pour former le premier produit coloré ;
(c) ajouter la solution d'arrêt au mélange réactionnel dans le récipient de réaction pour produire le deuxième et le troisième produits colorés ; et,
(d) mesurer les absorbances par le biais d'analyses spectrographiques à la première, la deuxième et la troisième longueurs d'onde, une réaction positive étant détectée par une absorbance à la seconde longueur d'onde, des réactions qui ne présentent aucune absorbance à la première ou à la seconde longueur d'onde, mais qui présentent une absorbance à la troisième longueur d'onde étant évaluées comme étant négatives, et toutes les réactions ne présentant aucune absorbance à la troisième longueur d'onde étant évaluées comme de fausses réactions négatives.

67. Utilisation d'un dosage par méthode immunoenzymatique pour distinguer une réaction négative d'une fausse réaction négative dans le dosage par méthode immunoenzymatique et dans laquelle le dosage par méthode immunoenzymatique est destiné à détecter de manière colorimétrique un anticorps d'un certain type, dans lequel une quantité d'un analyte est adsorbée sur un support solide dans un récipient de réaction ; un conjugué est formé entre un réactif immunologique et une peroxydase ; le conjugué est mélangé à un échantillon à tester à la recherche de l'anticorps, l'anticorps se lie à l'analyte et au conjugué afin de former un complexe immunologique en phase solide ; et la quantité de l'anticorps est déterminée en mesurant la réaction du complexe immunologique avec un substrat chromogène oxydable par un peroxyde et la peroxydase, comprenant les étapes consistant à :
(a) fournir une solution liquide comprenant le substrat chromogène, le peroxyde, et un colorant indicateur de pH, dans laquelle le substrat chromogène est la TMB ou l'OPD et est oxydable en un premier produit coloré présentant une absorbance à une première longueur d'onde lorsqu'un mélange réactionnel est formé entre la solution liquide et le complexe immunologique et est encore oxydable formant un deuxième produit coloré à partir du premier produit coloré présentant une absorbance à une deuxième longueur d'onde lorsqu'une solution d'arrêt est ajoutée dans le récipient de réaction et le colorant indicateur de pH produit un troisième produit coloré qui est détectable à l'oeil et qui présente une absorbance à une troisième longueur d'onde lorsqu'une solution d'arrêt est ajoutée dans le récipient de réaction contenant la solution liquide ;
(b) mélanger la solution liquide au complexe immunologique dans le récipient de réaction afin de former un mélange réactionnel qui oxyde le substrat chromogène pour former le premier produit coloré ;
(c) ajouter la solution d'arrêt au mélange réactionnel dans le récipient de réaction pour produire le deuxième et le troisième produits colorés ; et,
(d) mesurer les absorbances par le biais d'analyses spectrographiques à la première, la deuxième et la troisième longueurs d'onde, une réaction positive étant détectée par une absorbance à la deuxième longueur d'onde, des réactions qui ne présentent aucune absorbance à la première ou à la deuxième longueur d'onde, mais qui présentent une absorbance à la troisième longueur d'onde étant évaluées comme étant négatives, et toutes les réactions ne présentant aucune absorbance à la troisième longueur d'onde étant évaluées comme de fausses réactions négatives.
